# EUROPÄISCHE PATENTSCHRIFT

(11) **EP 4 310 571 B1**
(45) Veröffentlichungstag und Bekanntmachung des Hinweises auf die Patenterteilung: **15.01.2025**
(21) Anmeldenummer: 22186407.7
(22) Anmeldetag: 22.07.2022
(51) Int. Cl.: G02B 21/26, G02B 21/24

(54) **KIPPVORRICHTUNG ZUR MIKROSKOPIE**
TILTING DEVICE FOR MICROSCOPY
DISPOSITIF D'INCLINAISON DESTINÉ À LA MICROSCOPIE

(43) Veröffentlichungstag der Anmeldung: 24.01.2024
(73) Patentinhaber: ibidi GmbH, 82166 Gräfelfing (DE)
(72) Erfinder: Kahl, Valentin, 82152 Martinsried (DE)
(74) Vertreter: Grünecker Patent- und Rechtsanwälte PartG mbB

(56) Entgegenhaltungen:
- EP-A1- 1 397 483
- WO-A2-2014/210536
- DE-C- 579 789
- DE-C- 608 804

## Beschreibung

Die Erfindung betrifft eine Kippvorrichtung zur Mikroskopie und ein Mikroskop umfassend die Kippvorrichtung, sowie ein zugehöriges Verfahren und eine Verwendung.

Es gibt unterschiedlich ausgebildete Probenträger für lebende Zellen, in denen die Zellen kultiviert und gleichzeitig mikroskopiert werden können. Auch gibt es Probenträger, in denen Zellen unter Fluss kultiviert werden können, oder das Medium, in dem die Zellen kultiviert werden, einfach ausgetauscht werden kann. Um einen solchen Fluss in einem Probenträger zu realisieren, besitzen Probenträger typischerweise ein darin ausgebildetes Kanalsystem, das mindestens einen Zulauf und einen Ablauf umfasst und ansonsten geschlossen ist. Probenträger mit Kanalsystemen sind beispielsweise aus EP 1 458 483 A2, EP 1 397 483 A1 und EP 4 030 216 A1 bekannt. Derartige Probenträger sind in der Regel für mikroskopische Untersuchungen konzipiert, sodass Zellen, die in dem Kanalsystem kultiviert werden, mikroskopiert werden können.

Um das Medium, insbesondere eine Flüssigkeit, in dem Kanalsystem auszutauschen, können ein Pumpsystem oder externe Reservoire eingesetzt werden, an die der Probenträger beispielsweise über Schläuche angeschlossen wird. Ein derartiges Pumpsystem ist zum Beispiel in EP 1 944 084 A1 offenbart. Im Fall externer Reservoire kann über unterschiedliche Füllstände der Reservoire und das Prinzip der kommunizierenden Röhren ein Flüssigkeitsaustausch unter Verwendung ausschließlich passiver Komponenten erreicht werden. Beide Möglichkeiten bedürfen jedoch einer zusätzlichen Vorrichtung neben dem Probenträger selbst, um einen Fluss innerhalb des Kanalsystems zum Austausch des Medium zu bewerkstelligen. Als Alternative kann der Probenträger selbst gekippt werden, um einen Fluss innerhalb des Kanalsystems mittels Gravitation zu erzeugen. Ein Nachteil dieser Lösung tritt bei gleichzeitiger Kippung und Mikroskopie zutage. Durch die Kippung verändert sich der Abstand zwischen einem Objektiv eines Mikroskops und dem Probenträger, beziehungsweise dem Objektiv und dem zu mikroskopierenden Bereich innerhalb des Probenträgers, sodass das Objektiv entsprechend nachfokussiert werden muss.

Eine Kippvorrichtung zur Mikroskopie ist aus der Druckschrift DE 608 804 C bekannt, bei der das Objektiv mit einem Spiegelsystem in zwei gegeneinander verschwenkten Lagen am Mikroskop befestigen lässt.

In Anbetracht der genannten Nachteile ist es eine Aufgabe der vorliegenden Erfindung, eine Kippvorrichtung zur Mikroskopie, ein Mikroskop, sowie ein zugehöriges Verfahren und eine Verwendung bereitzustellen, die es ermöglichen, Mikroskopie für einen Probenträger, in dem ein Fluss erzeugt werden oder vorhanden sein soll, zu verbessern. Diese Aufgabe wird durch die Kippvorrichtung nach Anspruch 1, das Mikroskop nach Anspruch 9, das Verfahren Anspruch 12, sowie die Verwendung nach Anspruch 14 gelöst. Weiterbildungen finden sich in den jeweiligen Unteransprüchen.

Erfindungsgemäß wird eine Kippvorrichtung zur Mikroskopie bereitgestellt, umfassend
einen Probenträgerhalter und
eine optische Komponente mit einer optischen Achse,
wobei die optische Achse zu einer Grundseite des Probenträgerhalters ausgerichtet ist,
wobei der Probenträgerhalter und die optische Komponente jeweils kippbar gelagert sind, und
wobei die Ausrichtung der optischen Achse bezüglich der Grundseite des Probenträgerhalters erhalten bleibt, wenn der Probenträgerhalter und die optische Komponente gekippt werden.

Werden der Probenträgerhalter und die optische Komponente gekippt, so bleibt in der Kippvorrichtung gemäß der vorliegenden Erfindung die Ausrichtung der optischen Achse der optischen Komponente bezüglich des Probenträgerhalters bei der Kippung erhalten. Hierdurch muss die optische Komponente nicht an die Kippung des Probenträgerhalters angepasst werden, was eine verbesserte Mikroskopie an einem Probenträger in einem gekippten Probenträgerhalter ermöglicht.

Eine Kippung wird um einen definierten Punkt oder eine definierte Achse durchgeführt. Die Kippung kann im Uhrzeigersinn oder entgegen dem Uhrzeigersinn erfolgen.

Ein Probenträgerhalter ist eine Vorrichtung, die ausgebildet ist, darin einen Probenträger zu haltern. Dabei weist der Probenträgerhalter eine Grundseite auf, die der optischen Komponente zugewandt ist. Der Probenträgerhalter kann insbesondere eine Grundseite mit einer Aussparung aufweisen, durch die hindurch mikroskopiert werden kann und wobei zwischen dem Probenträger und der optischen Komponente kein weiteres Element angeordnet ist. Alternativ kann der Probenträgerhalter auch nur ein seitlicher Rahmen sein, in den ein Probenträger beispielsweise durch Klemmen montiert wird und der den Probenträger teilweise oder vollständig umgibt.

Die Erfindung stellt zudem Kippvorrichtung für ein Mikroskop bereit, umfassend
einen Probenträgerhalter und
eine optische Komponente mit einer optischen Achse,
wobei die optische Achse zu einer Grundseite des Probenträgerhalters ausgerichtet ist,
wobei der Probenträgerhalter und die optische Komponente jeweils kippbar gelagert sind, und
wobei die Ausrichtung der optischen Achse bezüglich der Grundseite des Probenträgerhalters erhalten bleibt, wenn der Probenträgerhalter und die optische Komponente gekippt werden.

Die Grundseite des Probenträgerhalters kann parallel zu einer Unterseite eines in dem Probenträger gehalterten Probenträgers sein.

Dadurch ist die Ausrichtung des Probenträgers auch parallel zur Grundseite des Probenträgerhalters, die in der Kippvorrichtung als Bezugsgröße dient. Somit ist bei Mikroskopie keine optische Korrektur, die einen potentiellen Winkelversatz des Probenträgers, dem eigentlichen Objekt der Mikroskopie, gegenüber der Grundseite des Probenträgerhalters zu berücksichtigen. Eine zusätzliche Quelle für mögliche Abbildungsfehler wird daher vermieden.

Die optische Achse der optischen Komponente ist eine Symmetrielinie durch die optische Komponente, beispielsweise eine Rotations-Symmetrieachse der optischen Komponente. Im Fall von Spiegeln oder Linsen verläuft die optische Achse durch das jeweilige Krümmungszentrum und orthogonal zu den restlichen Symmetrieachsen dieser optischen Komponenten. Gleichzeitig definiert die optische Achse in der Strahlenoptik einen optischen Pfad, auf dem Licht durch ein optisches System propagiert.

Die optische Komponente kann unterhalb des Probenträgerhalters angeordnet sein.

Dadurch ist die Kippvorrichtung insbesondere für eine Verwendung im Zusammenhang mit einem inversen Mikroskop verwendbar.

Die Ausrichtung zwischen der optischen Achse und der Grundseite des Probenträgerhalters kann insbesondere senkrecht sein.

Diese spezielle Anordnung ermöglicht eine verbesserte Abbildung bei Mikroskopie unter Verwendung der Kippvorrichtung. Vor allem Koma, das durch schräg zur optischen Achse einfallende Lichtstrahlen als Kombination von Astigmatismus und sphärischen Aberrationen entsteht, wird durch eine senkrechte Ausrichtung zwischen der optischen Achse und der Grundseite des Probenträgerhalters unterdrückt.

Bei der optischen Komponente kann es sich um ein Objektiv und/oder einen Spiegel handeln.

Die meisten Mikroskope, insbesondere Durchlichtmikroskope wie inverse Mikroskope sind derart aufgebaut, dass eine Sammellinse, ein Objektiv, auf das zu mikroskopierende Objekt gerichtet ist. Bei inversen Mikroskopen werden Untersuchungen an einem Probenträger durchgeführt, indem das Objektiv auf dessen Grundseite gerichtet ist. Die Kippvorrichtung kann daher in ein existierendes Mikroskop integriert werden und damit zusätzlich die oben beschriebenen Vorteile bereitstellen.

Anstelle eines einzelnen Spiegels oder einer einzelnen Linse kann die optische Komponente auch mehreren Teilen bestehen, beispielsweise einer Doublet-Linse (wie zum Beispiel einem Achromat) oder einer Triplet-Linse. Ebenfalls möglich ist eine Kombination aus Spiegeln und Linsen.

Die Verwendung einer mehrkomponentigen optischen Komponente erlaubt eine höhere Flexibilität der Kippvorrichtung, beispielsweise um mehr mechanische Freiheitsgrade zu erhalten oder optische Korrekturen durchzuführen wie mit einer achromatischen Linse.

Insbesondere kann die optische Komponente sowohl ein Objektiv als auch einen Spiegel umfassen. In einer besonderen Konfiguration ist dabei ein Objektiv auf die Grundseite des Probenträgerhalters gerichtet und zwischen dem Probenträgerhalter und einem Spiegel angeordnet. Dabei lenkt der Spiegel Licht, das durch das Objektiv tritt, auf einen bestimmten Zielpunkt um. Bei einer Kippung des Probenträgerhalters vollführen das Objektiv und der Spiegel eine Kippung um eine gemeinsame Rotationsachse, sodass die relative Ausrichtung des Spiegels, des Objektivs und des Probenträgerhalters konstant bleibt. Die optische Achse entspricht hierbei dem optischen Pfad, der ausgehend vom Probenträger durch das Objektiv verläuft und vom Spiegel durch Reflexion umgelenkt wird. Erfolgt die Kippung der Kippvorrichtung um eine Achse, die senkrecht zur optischen Achse, zwischen Probenträgerhalter und Objektiv, sowie koaxial zur optischen Achse zwischen dem Spiegel und dem Zielpunkt liegt, so ändert sich der Zielpunkt aufgrund der Kippung nicht. Hierbei lenkt der Spiegel das Licht insbesondere in einem rechten Winkel um. Trotz der Kippung bleibt der optische Pfad zwischen dem Spiegel und dem Zielpunkt also unverändert.

Die Anordnung von Spiegel und Objektiv kann auch in umgekehrter Reichenfolge ausgebildet sein, wobei in diesem Fall lediglich der Spiegel als optische Komponente betrachtet werden kann. In dieser Konfiguration ist der Spiegel entlang des optischen Pfads zwischen dem Probenträgerhalter und dem Objektiv angeordnet, sodass er Licht aus Richtung des Probenträgerhalters umlenkt, sodass es auf das Objektiv fällt. Hierbei ist das Objektiv fest montiert und der Spiegel und der Probenträgerhalter sind kippbar gelagert führen eine Kippbewegung aus. Erfolgt die Kippung der Kippvorrichtung um eine Achse, die senkrecht zur optischen Achse, zwischen Probenträgerhalter und Spiegel, sowie koaxial zur optischen Achse zwischen dem Spiegel und dem Objektiv liegt, und wird das Licht vom Spiegel in einem rechten Winkel umgelenkt, dann ändern sich ein Auftreffpunkt und ein Auftreffwinkel des Lichts auf dem Objektiv aufgrund der Kippung nicht. Trotz der Kippung bleibt der optische Pfad zwischen dem Spiegel und dem Objektiv also unverändert.

Ein Vorteil dieser zweitgenannten Konfiguration besteht darin, dass das Objektiv fest montiert ist und sich bei einer Kippung der Kippvorrichtung nicht bewegt. Dies ermöglicht eine einfachere Bauweise und insbesondere eine einfachere Integration in ein bestehendes Mikroskop, das bereits ein Objektiv umfasst. Weiterhin können Abbildungsfehler, die durch eine zeitliche Variation der Objektivposition entstehen, verringert werden, was in einer verbesserten Abbildungsqualität resultieren kann.

Der Probenträgerhalter und die optische Komponente der Kippvorrichtung können jeweils mit einem Gelenk verbunden sein und um das jeweilige Gelenk jeweils kippbar gelagert sein.

Dies bedeutet, dass die optische Komponente und der Probenträger für sich einzeln kippbar gelagert sind, bei einer Kippung aber die besagte Ausrichtung erhalten bleibt. Hierzu können beide Komponenten unabhängig voneinander kippbar und um eine eigene Kippachse gelagert sein. Dabei sind die Kippachse der optischen Komponente und die Kippachse des Probenträgerhalters koaxial. Dies ermöglicht eine größere Flexibilität und zahlreiche Realisierungsoptionen und Möglichkeiten der Integration einer solchen Kippvorrichtung in ein Mikroskop.

Ein Gelenk stellt eine mechanisch etablierte und zuverlässige Möglichkeit dar, um Kippungen und Drehungen bestimmter Elemente zu realisieren.

Der Probenträgerhalter und die optische Komponente können mit demselben Gelenk verbunden sein und um dieses Gelenk kippbar gelagert sein.

Eine solche Konfiguration stellt eine einfache und verlässliche Anordnung dar, um eine Kippung der optischen Komponente und des Probenträgerhalters zu realisieren, bei der die beschriebene senkrechte Ausrichtung zwischen der optischen Achse und der Grundseite des Probenträgerhalters im Fall einer Kippung erhalten bleibt. Insbesondere wird hierdurch bei einer Kippung eine ungewollte relative Bewegung zwischen der optischen Komponente und dem Probenträgerhalter verhindert, da beide Komponenten gemeinsam gekippt werden.

Typische Beispiele für ein Gelenk umfassen hierbei Kugelgelenke und Drehgelenke oder Scharniergelenke. Während bei einem Drehgelenk Rotationen lediglich um eine Achse stattfinden können, erlaubt ein Kugelgelenk eine Rotation um eine Vielzahl von Achsen, die in einer Ebene angeordnet sind. Ein Kugelgelenk bietet daher eine größere Flexibilität hinsichtlich der Kippachse. Gleichzeitig ermöglicht ein Drehgelenk eine Rotation um eine wohldefinierte Achse und bietet daher eine bessere Präzision und Stabilität. Für die oben beschriebenen, besonderen Konfigurationen für den Probenträgerhalter und die optische Komponente bietet sich ein Drehgelenk an, da es eine Rotation nur um eine wohldefinierte Achse erlaubt.

Der Probenträgerhalter und die optische Komponente können starr miteinander verbunden sein.

Eine starre Verbindung zwischen dem Probenträgerhalter und der optischen Komponente stellt eine einfache Möglichkeit dar, dass bei einer Kippung der beiden genannten Komponenten die senkrechte Ausrichtung der optischen Achse der optischen Komponente gegenüber der Grundseite des Probenträgerhalters erhalten bleibt. Zudem zeichnet sich eine starre Verbindung durch eine hohe Robustheit aus, sodass die relative Ausrichtung besagter Komponenten besonders stabil bleibt. Dies ist im Zusammenhang mit der in der Mikroskopie benötigten Präzision vorteilhaft, weil es nicht zu einer relativen Bewegung zwischen der optischen Komponente und dem Probenträgerhalter kommen kann, die zu, gegebenenfalls zeitlich variierenden, Abbildungsfehlern führen könnte.

Die Kippvorrichtung kann derart ausgebildet sein, dass der Probenträgerhalter und die optische Komponente um eine Kippachse gekippt werden, die senkrecht zur optischen Achse ausgerichtet ist.

Mit anderen Worten kann sich die Kippachse in einer Ebene befinden, die senkrecht zur optischen Achse liegt und die Kippung wird um diese Achse durchgeführt.

Die Einschränkung, dass die Kippachse senkrecht zur optischen Achse ausgerichtet ist, ermöglicht dennoch eine hohe Flexibilität hinsichtlich der Kippung. Beispielsweise kann man um die Kippvorrichtung ein Koordinatensystem definieren, in dem die optische Achse entlang der Z-Achse ausgerichtet ist. Die Kippung erfolgt in diesem Fall um eine Kippachse senkrecht zur Z-Achse, also beispielsweise um die X-Achse, die Y-Achse oder eine beliebige Superposition dieser beiden Achsen. Im letztgenannten Fall besitzt die Kippvorrichtung die höchstmögliche Zahl an Freiheitsgraden für die Kippung, weil eine Rotation um die Z-Achse keiner Kippung entspricht.

Die Kippvorrichtung kann derart ausgebildet sein, dass der Probenträgerhalter und die optische Komponente der Kippvorrichtung bezüglich einer horizontalen Position des Probenträgerhalters um einen Kippwinkel zwischen 0 Grad und 1 Grad, insbesondere zwischen 0 Grad und 20 Grad gekippt werden können.

Insbesondere kann die Kippvorrichtung derart ausgebildet sein, dass der Probenträgerhalter und die optische Komponente der Kippvorrichtung eine Pendelkreisbewegung ausführen können. Hierunter wird eine zeitabhängige Kippbewegung verstanden, bei der ein zur optischen Achse paralleler Vektor eine kreisförmige elliptische Bahn beschreibt. Diese Bewegung wird durch eine zeitabhängige Superposition einer Kippung um die X-Achse und einer Kippung um die Y-Achse erzeugt..

Je nach Spanne der erreichbaren Kippwinkel erlaubt die Kippvorrichtung unterschiedliche Anwendungen. Für eine geringe Spanne zwischen 0 Grad und 1 Grad kann eine präzise Kippung vorgenommen werden, für Untersuchungen, in denen nur geringe aber exakte Kippwinkel benötigt werden. Wie eingangs beschrieben, kann die Kippvorrichtung dafür benutzt werden, in einem Probenträger Flüssigkeiten zu transportieren. Die Flussgeschwindigkeit kann auf diese Weise besonders gering und genau eingestellt werden, sodass Zellen unter wohldefinierten Bedingungen untersucht werden können. Eine größere Bandbreite an Kippwinkeln, beispielsweise zwischen 0 Grad und 20 Grad, erhöht hingegen die Vielseitigkeit möglicher durchführbarer Untersuchungen. Gleichzeitig kann eine Flüssigkeit, beispielsweise ein Zellmedium zügig ausgetauscht werden. Bei einer Pendelkreisbewegung kann in einem Probenträger ein kreisförmiger Fluss erzeugt werden

Die Kippvorrichtung kann weiterhin eine Bewegungseinrichtung umfassen, wobei die Bewegungseinrichtung derart ausgebildet ist, dass sie die optische Komponente in einer Ebene parallel zur Grundseite des Probenträgerhalters bewegen kann, oder wobei die Bewegungseinrichtung derart ausgebildet ist, dass sie den Probenträgerhalter in einer Ebene senkrecht zur optischen Achse bewegen kann.

In diesem Fall kann die Bewegungseinrichtung den Probenträgerhalter bewegen, während die optische Komponente fest montiert ist. Alternativ kann die Bewegungseinrichtung die optische Komponente bewegen, während der Probenträgerhalter fest montiert ist.

Die Bewegungseinrichtung kann weiterhin derart ausgebildet sein, dass sie die optische Komponente senkrecht zur Grundseite des Probenträgerhalters, beziehungsweise parallel zur optischen Achse der optischen Komponente, bewegen kann, oder dass sie den Probenträgerhalter parallel zur optischen Achse der optischen Komponente bewegen kann.

Auch in diesem Fall kann die Bewegungseinrichtung den Probenträgerhalter bewegen, während die optische Komponente fest montiert ist. Alternativ kann die Bewegungseinrichtung die optische Komponente bewegen, während der Probenträgerhalter fest montiert ist.

Diese Ausführungsform trifft nicht auf eine Kippvorrichtung zu, in der die optische Komponente und der Probenträgerhalter starr miteinander verbunden sind.

Durch wird eine relative Bewegung zwischen der optischen Komponente und dem Probenträgerhalter in einer Ebene parallel zur Grundseite des Probenträgerhalters wird erreicht, dass sich ein Abstand zwischen der optischen Komponente und dem Probenträgerhalter oder die senkrechte Ausrichtung zwischen der optischen Achse und der Grundseite des Probenträgerhalters nicht ändern. Gleichzeitig ist es möglich, einen in den Probenträgerhalter eingebrachten Probenträger an unterschiedlichen Stellen zu untersuchen, insbesondere auch während der Durchführung einer Kippbewegung. Eine Anpassung der optischen Komponente, insbesondere eine Nachfokussierung eines Objektivs ist in diesem Fall nicht notwendig.

Darüber hinaus erlaubt eine relative Bewegung zwischen dem Probenträgerhalter und der optischen Komponente parallel zur optischen Achse zusätzlich, dass ein Fokus der optischen Komponente an unterschiedlichen Tiefen innerhalb eines Probenträgers liegen kann. Somit ist zusammen neben der oben beschriebenen horizontalen relativen Bewegung zwischen dem Probenträgerhalter und der optischen Komponente nun eine vollständige dreidimensionale Untersuchung eines Probenträgers möglich. Die eingangs beschriebenen Vorteile der Kippvorrichtung treffen in der gleichen Art und Weise zu.

Die Bewegungseinrichtung kann hierbei beispielsweise manuell steuerbar sein. Dazu kann beispielsweise der Probenträgerhalter oder die optische Komponente mit einer Translationsplattform (englische Bezeichnung: *"translation stage"*) verbunden sein. Mit einer entsprechenden Stellschraube der Translationsplattform kann manuell eine Bewegung der verbundenen Komponente erreicht werden. Die Bewegungseinrichtung kann weiterhin eine Feststellschraube oder ein vergleichbares Element umfassen, mit der die Bewegungseinrichtung nach vorgenommener Einstellung fixiert werden kann, sodass eine weitere relative Bewegung zwischen der optischen Komponente und dem Probenträgerhalter ausgeschlossen ist.

Herkömmliche Translationsplattformen erlauben hierbei typischerweise eine Präzision der Einstellung in Bereich von wenigen zehn Mikrometern, was für die meisten Anwendungen in der Mikroskopie ausreichend ist.

Gleichsam kann die Bewegungseinrichtung auch motorisiert ausgebildet sein, beispielsweise in Form einer motorisierten Translationsplattform ("*motorized translation stage"*). Davon abgesehen treffen die gleichen Überlegungen wie für eine manuelle Ausführungsform zu. Es versteht sich, dass im Fall einer motorisierten Bewegungseinrichtung eine zugehörige Steuerung benötigt wird. Diese wird nachfolgend beschrieben.

Eine motorisierte Bewegungseinrichtung birgt mehrere Vorteile. Zum einen sind die Auflösung und die Präzision der Einstellung typischerweise höher als in einer manuellen Ausführung. Darüber hinaus kann die motorisierte Bewegungseinrichtung automatisiert und extern gesteuert werden, beispielsweise über eine externe Kontrolleinrichtung wie einen Computer. Dies ermöglicht neben einer präzisen Kontrolle der relativen Position zwischen der optischen Komponente und dem Probenträgerhalter auch zeitlich gesteuerte und automatisiert ablaufende Bewegungssteuerung.

Die Kippvorrichtung umfasst weiterhin eine Neigungseinrichtung mit einem Motor,
wobei der Motor mit der optischen Komponente und/oder dem Probenträgerhalter verbunden ist, und
wobei der Motor derart ausgebildet ist, dass er den Probenträgerhalter und die optische Komponente kippt.

Diese Neigungseinrichtung erlaubt es, den Kippwinkel automatisiert einzustellen, insbesondere mittels einer externen Kontrolleinrichtung wie einem Computer. Im Allgemeinen erlaubt dies eine höhere Präzision als bei einer manuellen Einstellung. Weiterhin kann die mikroskopische Beobachtung simultan mit der Kippung erfolgen und muss dafür nicht unterbrochen werden. Insbesondere kann die Ausführung der Neigungseinrichtung, insbesondere die Form des Motors davon abhängen, welche Kippwinkel erreicht werden sollen. Bei sehr geringen Kippwinkeln im Bereich von rund 1 Grad oder weniger bietet sich ein Motor in Form eines piezoelektrischen Aktors an. Diese ermöglichen eine Einstellung des Winkels mit sehr hoher Präzision und Reproduzierbarkeit. Die für den piezoelektrischen Aktor benötigte elektrische Spannung kann von der beschriebenen externen Kontrolleinrichtung bereitgestellt werden.

Für größere Winkel ist ein piezogetriebener Aktor allerdings eher ungeeignet, weil ein dafür benötigter Hub nicht erreicht werden kann. Hier bietet sich stattdessen beispielsweise ein entsprechend ausgebildeter Elektromotor beziehungsweise ein Drehmotor an.

Die Neigungseinrichtung ist weiterhin derart ausgebildet, dass sie eine kontinuierliche Kippbewegung, insbesondere eine wippende Bewegung der Kippvorrichtung erzeugt. Eine wippende Bewegung ist eine andauernde Kippbewegung, bei der sich in wohldefinierten zeitlichen Abständen eine Richtung der Kippbewegung ändert. Um dies zu erreichen, muss die Neigungseinrichtung eine zeitlich abhängige Winkelgeschwindigkeit erlauben, bei der insbesondere auch ein Vorzeichenwechsel der Winkelgeschwindigkeit möglich ist.

Bestimmte Zelltypen müssen unter einem kontinuierlichen Fluss kultiviert werden. Hierbei stellt eine kippende Bewegung der Kippvorrichtung und damit auch eines gehalterten Probenträgers eine Möglichkeit dar, diesen kontinuierlichen Fluss ohne zusätzliche Komponenten zu erzeugen. Durch die Änderung der Richtung der Kippbewegung kann eine Flüssigkeit im Probenträger ständig in Bewegung gehalten werden, ohne dass dabei zusätzliche Flüssigkeit nachgefüllt werden müsste.

Darüber hinaus ist es möglich, neben der motorgetriebenen Neigungseinrichtung auch eine zusätzliche manuelle Einstellung zuzulassen. Hierfür kann die Kippvorrichtung beispielsweise eine Feststellschraube umfassen, die von Hand gelöst und wieder festgezogen werden kann. Die hat beispielsweise den Vorteil, dass eine große Einstellung des Kippwinkels von Hand vorgenommen werden kann, während die präzisere Einstellung motorisiert vorgenommen wird. Dadurch muss der Motor nur eine geringere Spanne an Kippwinkeln bereitstellen können.

Die Kippvorrichtung kann weiterhin einen Neigungssensor und eine Steuerungseinrichtung umfassen, wobei der Neigungssensor derart ausgebildet ist, dass er einen Wert eines Kippwinkels der Kippvorrichtung erfassen und ausgeben kann, und wobei die Steuerungseinrichtung derart ausgebildet ist, den von dem Neigungssensor ausgegebenen Wert zu empfangen und aufgrund des empfangenen Werts und eines vorgegebenen Zielwerts ein Steuerungssignal an die Neigungsvorrichtung zu übertragen, sodass die Neigungsvorrichtung den Kippwinkel auf den vorgegebenen Wert einstellt.

Diese Ausführungsform mit einem Neigungssensor und einer Steuerungseinrichtung erlaubt zum einen eine automatisierte Einstellung des Kippwinkels, wobei in dieser Hinsicht die gleichen Vorteile zutreffen wie bei einer Automatisierung der Bewegungseinrichtung. Darüber hinaus ermöglichen diese zusätzlichen Merkmale auch einen Feedback-Mechanismus, mit dem der Kippwinkel auf einen bestimmten Wert stabilisiert werden kann. Dadurch lassen sich Untersuchungen auch über lange Zeiträume und unter besonders stabilen und/oder wohldefinierten Bedingungen durchführen.

Für die Realisierung eines Neigungssensors sind unterschiedliche Ausführungsformen denkbar. In einer einfachen Form kann eine Winkelskala oder eine vergleichbare visuelle Markierung sichtbar an der Kippvorrichtung angebracht sein, an der der Kippwinkel ablesbar ist. Zudem kann an der Kippvorrichtung ein Neigungssensor angebracht sein, der den Wert des Kippwinkels über beispielsweise drei senkrecht zueinander stehende Beschleunigungssensoren oder mittels eines kapazitiven Flüssigkeitssensors oder eines magnetoresistiven Sensors erfassen und elektronisch ausgeben kann. Dieser ausgegebene Winkel kann dann von der externen Kontrolleinrichtung angezeigt oder anderweitig ausgegeben und/oder verwendet werden. Insbesondere kann der Wert des Kippwinkels an die Steuerungseinrichtung übertragen werden. Diese Übertragung kann dabei beispielsweise über Funk, Bluetooth, WLAN, RFID, LAN oder andere bekannte Übertragungsmechanismen stattfinden.

Die Steuerungseinrichtung kann den vom Neigungssensor ausgegebenen Wert für den Kippwinkel empfangen. Dabei kann die Steuerungseinrichtung einen Prozessor umfassen, um auf Grundlage des empfangenen Werts und eines vorgegebenen Zielwerts für den Kippwinkel das Steuerungssignal zu ermitteln oder zu berechnen. Dieses Steuerungssignal kann anschließend von der Steuerungseinrichtung an die Neigungseinrichtung übertragen werden, wobei für diese Übertragung die gleichen Möglichkeiten wie zwischen dem Neigungssensor und der Steuerungseinrichtung zur Verfügung stehen.

Die Neigungseinrichtung kann das Steuerungssignal empfangen und darauf basierend den Motor derart kontrollieren, dass der Kippwinkel an den vorgegebenen Zielwert angepasst wird.

Bei der Steuerungseinrichtung kann es sich auch um die beschriebene externe Kontrolleinrichtung handeln, die neben der Neigungseinrichtung auch die Steuerungseinrichtung umfasst. Dabei kann die externe Kontrolleinrichtung einen oder mehrere Prozessoren umfassen, um die Steuerungssignale für die zugehörigen Einrichtungen zu ermitteln.

Die Kippvorrichtung kann weiterhin eine Ausgleichsoptik, beispielsweise einen bewegbaren Spiegel, umfassen.

Die Ausgleichsoptik kann dabei derart ausgebildet sein, dass sie die Bewegung infolge der Kippung der Kippvorrichtung derart kompensiert, dass die ein Auftreffpunkt und/oder ein Auftreffwinkel von Licht, das durch den Probenträgerhalter und die optische Komponente tritt, auch bei einer Kippung unverändert bleiben.

Der bewegbare Spiegel kann ein Spiegel sein, der auf einem herkömmlichen motorisierten Spiegelhalter ("*motorized mirror mount*") montiert ist und über ein entsprechendes elektrisches Signal bewegbar ist. Dabei kann das elektrische Signal beispielsweise von der externen Kontrolleinrichtung bereitgestellt werden.

Die zusätzliche Ausgleichsoptik vereinfacht unter anderem die Integration der Kippvorrichtung in ein bestehendes, herkömmliches Mikroskop. So umfasst ein Mikroskop typischerweise neben einem Objektiv zumindest einen oder mehrere Umlenkspiegel und ein Okular, mit dem ein virtuelles Bild auf einen Sensor oder das menschliche Auge abgebildet wird. Die beschriebene Kippvorrichtung kann nun beispielsweise in ein solches Mikroskop integriert werden, indem das Objektiv und ein Objekttisch durch die beschriebene Kippvorrichtung ersetzt werden. Bei der Kippvorrichtung kann es sich folglich um ein Austauschmodul handeln, mit dem ein herkömmliches Mikroskop umgerüstet werden kann. Infolge einer Kippung verändert sich der optische Pfad von Licht im Mikroskop. Um die Bildgebung dadurch im allgemeinen Fall nicht zu beeinflussen, müssen der Auftreffpunkt und der Auftreffwinkel von Licht, das durch das Objektiv tritt, infolge der Kippung unverändert bleiben. Dies kann durch die beschriebene Ausgleichsoptik erreicht werden.

Weiterhin beinhaltet die vorliegende Erfindung ein Mikroskop umfassend eine zuvor beschriebene Kippvorrichtung, ein Stativ und ein Abbildungssystem mit einer Abbildungslinse, insbesondere einem Okular.

Durch den Einsatz einer beschriebenen Kippvorrichtung in einem Mikroskop ergeben sich auch die im Zusammenhang mit der Kippvorrichtung genannten Vorteile.

Im Zusammenhang mit der Kultivierung und Beobachtung von Zellen in dafür vorgesehenen Probenträgern wird typischerweise inverse Mikroskope bevorzugt verwendet. Dies liegt daran, dass die Zellen in der Regel an einem Boden des Probenträgers angeordnet sind. In einem inversen Mikroskop ist das Objektiv von unten auf das zu untersuchende Objekt gerichtet, sodass dadurch das Objektiv besonders nahe an die Zellen herangebracht werden kann. Dies ermöglicht eine höhere optische Auflösung. Allerdings kann die hierin beschriebene Kippvorrichtung auch in einem Mikroskop zum Einsatz kommen, bei dem das Objektiv von oben auf den Probenträger gerichtet ist, weil sich der grundsätzliche Aufbau der beiden genannten Mikroskoparten lediglich in der Anordnung der bildgebenden Komponenten unterscheidet, nicht aber die Komponenten selbst.

Das Abbildungssystem umfasst insbesondere ein Okular, das ein betrachtbares Bild erzeugt. Darüber hinaus können noch weitere optische Elemente von dem Abbildungssystem umfasst sein, beispielsweise einer oder mehrere Spiegel oder ein Prisma zur Umlenkung des Lichts. Das Okular selbst kann aus einer oder mehreren Linsen aufgebaut sein. Es ist möglich, das Bild durch das Okular erzeugte Bild mit dem menschlichen Auge zu betrachten.

Die Erfindung stellt weiterhin ein Mikroskop bereit, umfassend einen Probenträgerhalter, und eine optische Komponente mit einer optischen Achse, ein Stativ und ein Abbildungssystem mit einer Abbildungslinse, insbesondere einem Okular, wobei die optische Achse zu einer Grundseite des Probenträgerhalters ausgerichtet ist, wobei der Probenträgerhalter und die optische Komponente kippbar gelagert sind, wobei die Ausrichtung der optischen Achse bezüglich der Grundseite des Probenträgerhalters erhalten bleibt, wenn der Probenträgerhalter und die optische Komponente gekippt werden, und wobei die Abbildungslinse zwischen der optischen Komponente und der bildgebenden Komponente angeordnet ist.

Das Abbildungssystem des Mikroskops kann weiterhin einen oder mehrere Spiegel, insbesondere bewegbare Spiegel, umfassen, wobei die Spiegel mechanisch gekoppelt oder individuell elektronisch ansteuerbar sind. Das Abbildungssystem kann eine bildgebende Komponente, insbesondere eine Kamera mit einem bildgebenden Sensor umfassen.

Eine bildgebende Komponente in Form einer Kamera erlaubt eine automatisierte und gegebenenfalls kontinuierliche Aufnahme von Bilddaten mit dem Mikroskop. Diese Bilddaten können direkt ausgewertet werden, beispielsweise von einem dafür eingerichteten Prozessor.

Die Spiegel dienen vorwiegend der Übertragung eines Bildes vom Objektiv auf die bildgebende Komponente. Somit können die Spiegel dafür vorgesehen sein, das Licht vom Objektiv auf die bildgebende Komponente umzulenken, es handelt sich also um sogenannte Umlenkspiegel. Die mechanische Kopplung der Spiegel oder die elektronische Ansteuerbarkeit erfüllen dabei den Zweck, den optischen Pfad des Lichts, der sich infolge einer Kippung der Kippvorrichtung ändert, derart anzupassen, dass ein Auftreffpunkt und Auftreffwinkel auf die bildgebende Komponente dabei unverändert bleiben.

Die mechanische Kopplung kann dabei zwischen zwei oder mehreren Spiegeln ausgebildet sein, sodass diese gekoppelten Spiegel eine korrelierte Bewegung ausführen. Beispielsweise können die zwei oder mehreren Spiegeln über ein mechanisches Element direkt miteinander verbunden sein.

Mindestens einer der Spiegel kann auch auf einem motorisierten Spiegelhalter montiert sein, der die Ausrichtung des Spiegels einstellen kann. Der Motor kann dabei insbesondere von einer dafür vorgesehenen Vorrichtung verbunden sein und kontrolliert werden. Dabei kann es sich beispielsweise um die oben beschriebene externe Kontrolleinrichtung handeln, die auch die Neigungseinrichtung und die Steuereinrichtung kontrollieren kann. Eine individuelle Ansteuerung der Spiegel des Mikroskops erlaubt eine optimale Kontrolle des optischen Pfads durch das Mikroskop und daher eine präzise Anpassung der Ausrichtung der Spiegel, sodass die Abbildung infolge einer Kippung nicht beeinflusst wird.

Weiterhin kann das Mikroskop eine Anzeigevorrichtung, insbesondere einen Monitor, umfassen. Dadurch kann das von der bildgebenden Komponente erzeugte Bild angezeigt werden.

Die Abbildungslinse kann entlang ihrer optischen Achsen beweglich montiert sein, oder eine veränderliche Brennweite besitzen, wobei eine Bewegung der Abbildungslinse entlang ihrer optischen Achse und/oder die Brennweite individuell steuerbar ist/sind.

Abhängig von der Lage der Drehachse bei einer Kippung der Kippvorrichtung kann mit dem Verlauf des optischen Pfads auch dessen Länge verändern, was sich durch die zuvor beschriebenen Spiegel gegebenenfalls nicht kompensieren lässt. Dies hat zur Folge, dass die bildgebende Komponente nicht mehr in der Fokusebene der Abbildungslinse liegt und ein unscharfes Bild entsteht. Durch eine Änderung der Brennweite oder der Position der Abbildungslinse kann dessen Fokusebene (Bildebene) derart eingestellt werden, dass sie stets auf der bildgebenden Komponente liegt und ein scharfes Bild entsteht.

Die Abbildungslinse kann beweglich montiert sein, insbesondere in einer Schiene, sodass lediglich eine Bewegung entlang der optischen Achse der Abbildungslinse möglich ist. Damit sind zusammen mit der Ausgleichsoptik und/oder den Spiegeln des Mikroskops ausreichend viele Freiheitsgrade vorhanden, um eine Veränderung des optischen Pfads infolge der Kippung auszugleichen, sodass die Abbildung nicht verändert wird. Eine Bewegung der Abbildungslinse senkrecht zu ihrer optischen Achse ist hierfür nicht zwingend notwendig.

Die Bewegung kann entweder manuell durchgeführt werden oder durch die externe Kontrolleinrichtung gesteuert werden. Hierbei gelten die gleichen Erwägungen wie in der beschriebenen Einstellung eines Kippwinkels der Kippvorrichtung mithilfe der Neigungseinrichtung.

Zusätzlich zu oder anstelle der beweglichen Montierung kann die Abbildungslinse zusätzlich eine veränderliche Brennweite besitzen, wie beispielsweise eine Varifokallinse ("*varifocal lens*"). Eine Varifokallinse umfasst dabei eine Mehrzahl von einzelnen Linsen, die relativ zueinander bewegt werden können, sodass sich die Gesamtbrennweite des Systems ändert. Diese Linse löst damit auch das technische Problem, einen veränderten Fokus infolge der Kippung der Kippvorrichtung zu kompensieren. Auch hier kann die Einstellung der Brennweite ebenfalls manuell oder über die externe Kontrollvorrichtung vorgenommen werden.

Die veränderliche Position und die veränderliche Brennweite der Abbildungslinse können auch kombiniert werden, beziehungsweise gemeinsam eingesetzt werden. Zudem kann das die Abbildungslinse, insbesondere ein Okular mehrlinsig ausgebildet sein. In diesem Fall können eine, mehrere oder alle der Linsen bewegbar angeordnet sein, und oder eine veränderliche Brennweite besitzen. Jeder der Positionen und Brennweiten der Linsen können dabei manuell und/oder durch die externe Kontrollvorrichtung gesteuert und eingestellt werden.

Die oben beschriebenen Mechanismen zur Anpassung der Anpassungslinse erlauben eine große Flexibilität des Mikroskops. In einer besonderen Ausführungsform ist es möglich, dass die optische Komponente ein Objektiv ist, das auf unendlich korrigiert ist. Dabei beträgt der Arbeitsabstand des Objektivs etwa dessen Brennweite, sodass die Lichtstrahlen nach dem Objektiv im Wesentlichen parallel verlaufen. Daher führt eine Abweichung in der Weglänge zwischen Objektiv und bildgebender Komponente infolge einer Kippung nicht zwingend zu einem anderen Fokus der Abbildungslinse. Es wird also weiterhin ein scharfes Bild auf der bildgebenden Komponente erzeugt und eine Anpassung der Abbildungslinse ist nicht nötig.

Das Mikroskop kann von einem Inkubationssystem umschlossen sein, welches eine Temperatur, Gaskonzentration und/oder Luftfeuchtigkeit zumindest an einem Ort der optischen Komponente und des Probenträgerhalters reguliert. Ein derartiges Inkubationssystem ist beispielsweise aus EP 2 148 921 A2 bekannt.

Ein Inkubationssystem kann dabei sehr präzise die Kultivierungsbedingungen für Zellen einstellen und kontrollieren. Das bedeutet, dass sowohl die Beobachtung als auch die Kultivierung der Zellen in einer gemeinsamen Vorrichtung durchgeführt werden können.

Das Inkubationssystem kann derart ausgebildet sein, dass es einen, insbesondere abgeschlossenen, Raum um das Mikroskop bildet. Am Mikroskop selbst müssen keine strukturellen Veränderungen vorgenommen werden, um es in die Inkubationsvorrichtung zu integrieren.

Die vorliegende Erfindung umfasst weiterhin ein Verfahren. Dabei umfasst das Verfahren die folgenden Schritte:
Bereitstellen einer zuvor beschriebenen Kippvorrichtung zur Mikroskopie oder eines zuvor beschriebenen Mikroskops;
Haltern eines Probenträgers in den Probenträgerhalter; und
Kippen des Probenträgerhalters,
wobei der Probenträger ein erstes Reservoir, ein zweites Reservoir und ein Kanal umfasst,
wobei das erste Reservoir über den Kanal mit dem zweiten Reservoir verbunden ist,
wobei durch das Kippen des Probenträgerhalters eine Flüssigkeit vom ersten Reservoir in das zweite Reservoir transportiert wird und wobei insbesondere durch den Transport der Flüssigkeit ein Fluss entsteht, und wobei Zellen unter diesem Fluss kultiviert werden.

Eine vollständige Durchführung dieses Verfahrens ermöglicht es, innerhalb eines Probenträgers eine Flüssigkeit ohne das Vorhandensein einer externen Vorrichtung, beispielsweise einer Pumpvorrichtung, sondern lediglich infolge einer Kippung des Probenträgers, zu transportieren. Dieser Flüssigkeitstransport erzeugt einen Fluss, unter dem Zellen in diesem Probenträger kultiviert werden. Beispielsweise ist ein solcher Fluss notwendig, um die Zellen mit Nährstoffen zu versorgen oder um benutzte Flüssigkeit auszutauschen. Darüber hinaus bietet das Verfahren weiterhin die Möglichkeit, den Probenträger auch während der Durchführung einer Kippung kontinuierlich zu mikroskopieren, weil sich der Arbeitsabstand und die Ausrichtung zwischen dem Probenträger und der optischen Komponente nicht ändern und die optische Komponente daher während der Mikroskopie nicht an den veränderten Fokus angepasst werden muss. Dies erlaubt eine effiziente Mikroskopie.

Ein Probenträger ist hierbei eine Plattform für die Untersuchung von Zellen. Der Probenträger kann insbesondere eine plane Unterseite besitzen, was für mikroskopische Untersuchungen durch diese Unterseite hindurch, vor allem im Fall inverser Mikroskopie, besonders von Vorteil ist. Weiterhin besteht der Probenträger vorzugsweise aus einem transparenten Material, beispielsweise aus Kunststoffen wie COC (Cyclo-Olefin Copolymer), COP (Cyclo-Olefin Polymer), PC (Polycarbonat), PS (Polystyrol), PE (Polyethylen), PMMA (Polymethymetacrylat) oder einen transparenten Thermoplast oder ein Elastomer oder einer Mischung davon.

Durch die Verwendung der genannten Materialien und Verfahren können die Probenträger kostengünstig, sowie in großer Stückzahl mit konstanter Qualität produziert werden. Dies liegt daran, dass der Spritzguss mit Kunststoffen ein etabliertes und verlässliches Verfahren ist und insbesondere im Fall der genannten Kunststoffe anwendbar ist. Die Verwendung eines transparenten Kunststoffs ist insbesondere vorteilhaft, um optische Untersuchungen im Probenträger vornehmen zu können, beispielsweise via Mikroskopie.

Das beschriebene Verfahren kann weiterhin mit einer Kippvorrichtung umfassend eine Neigungseinrichtung durchgeführt werden, wobei der Probenträger weiterhin einen Flüssigkeitssensor umfasst, und wobei der Flüssigkeitssensor einen Wert eines oder mehrerer von einem Flüssigkeitsstand, einer Flussgeschwindigkeit oder einer Flussrichtung innerhalb des in dem Probenträgerhalter gehalterten Probenträgers erfasst und ausgibt, und wobei die Steuerungseinrichtung von dem Sensor ausgegebenen Wert empfängt und aufgrund des empfangenen Werts ein Steuerungssignal an die Neigungseinrichtung überträgt, sodass die Neigungseinrichtung einen Kippwinkel der Kippvorrichtung einstellt, sodass eines oder mehrere von dem Flüssigkeitsstand, der Flussgeschwindigkeit oder der Flussrichtung innerhalb des Probenträgers an einen vordefinierten Zielwert angepasst werden.

Die Kultivierung von Zellen in einem Probenträger sollte vorzugsweise unter wohldefinierten Umgebungsbedingungen stattfinden, wozu insbesondere auch die genannten Parameter Flüssigkeitsstand, Flussgeschwindigkeit und Flussrichtung zählen. Diese Parameter können durch die Steuerungseinrichtung in Verbindung mit dem Flüssigkeitssensor präzise und stabil kontrolliert und eingestellt werden, sodass in dem Probenträger optimale und stabile Bedingungen für die Kultivierung von Zellen bereitgestellt werden können. Die Kontrolle der genannten Parameter kann dabei insbesondere automatisiert erfolgen, sodass der gesamte Kultivierungsprozess entsprechend auch automatisch ohne durchgehende Kontrolle durch einen menschlichen Operator ablaufen kann.

Zudem umfasst die vorliegende Erfindung eine Verwendung einer zuvor beschriebenen Kippvorrichtung zur Mikroskopie oder eines zuvor beschriebenen Mikroskops zum Transportieren einer Flüssigkeit innerhalb eines Probenträgers,
wobei der Probenträger in dem Probenträgerhalter gehaltert ist,
wobei der Probenträger ein erstes Reservoir, ein zweites Reservoir und ein Kanal umfasst,
wobei das erste Reservoir über den Kanal mit dem zweiten Reservoir verbunden ist,
wobei durch ein Kippen des Probenträgerhalters eine Flüssigkeit vom ersten Reservoir in das zweite Reservoir transportiert wird und wobei insbesondere durch den Transport der Flüssigkeit ein Fluss entsteht, und wobei Zellen unter diesem Fluss kultiviert werden.

Hiermit gehen die gleichen Vorteile einher wie beim vorstehend beschriebenen Verfahren. Dies betrifft insbesondere die Kultivierung von Zellen unter einem Fluss in einem Probenträger und die ermöglichte kontinuierliche Mikroskopie bei Kippung der Kippvorrichtung, weil der Fokus der optischen Komponente nicht korrigiert werden muss.

Weitere Merkmale und Vorteile werden nachfolgend anhand der beispielhaften Figuren erläutert. Dabei zeigen:
- Figuren 1A und 1B: einen schematischen Querschnitt in Frontansicht einer Kippvorrichtung gemäß einer ersten und einer zweiten Ausführungsform;
- Figur 2A: einen schematischen Querschnitt in Frontansicht einer Kippvorrichtung gemäß einer weiteren Ausführungsform;
- Figur 2B: einen schematischen Querschnitt in Seitenansicht einer Kippvorrichtung gemäß einer weiteren Ausführungsform;
- Figur 3A: eine schematische Kippvorrichtung umfassend eine Bewegungseinrichtung als Draufsicht;
- Figur 3B: eine schematische Kippvorrichtung umfassend eine Bewegungseinrichtung im Querschnitt;
- Figur 4: einen schematischen Querschnitt in Seitenansicht einer Kippvorrichtung gemäß einer weiteren Ausführungsform;Figur 5A ein schematisches Mikroskop gemäß einer ersten Ausführungsform im Querschnitt;
- Figur 5B: eine Detailansicht des Mikroskops in Figur 5A;
- Figur 5C: einen schematischen Querschnitt eines Mikroskops gemäß einer weiteren Ausführungsform;
- Figur 5D: ein schematisches Mikroskop gemäß einer weiteren Ausführungsform im Querschnitt;
- Figuren 6A: ein Flussdiagramm für ein Verfahren zur Mikroskopie; und
- Figuren 6B bis 6D: ein Verfahren zur Mikroskopie.

Im Folgenden und in den Figuren werden in den verschiedenen Ausführungsbeispielen, sofern nicht anders spezifiziert, die gleichen Bezugszeichen für gleiche oder entsprechende Elemente verwendet.

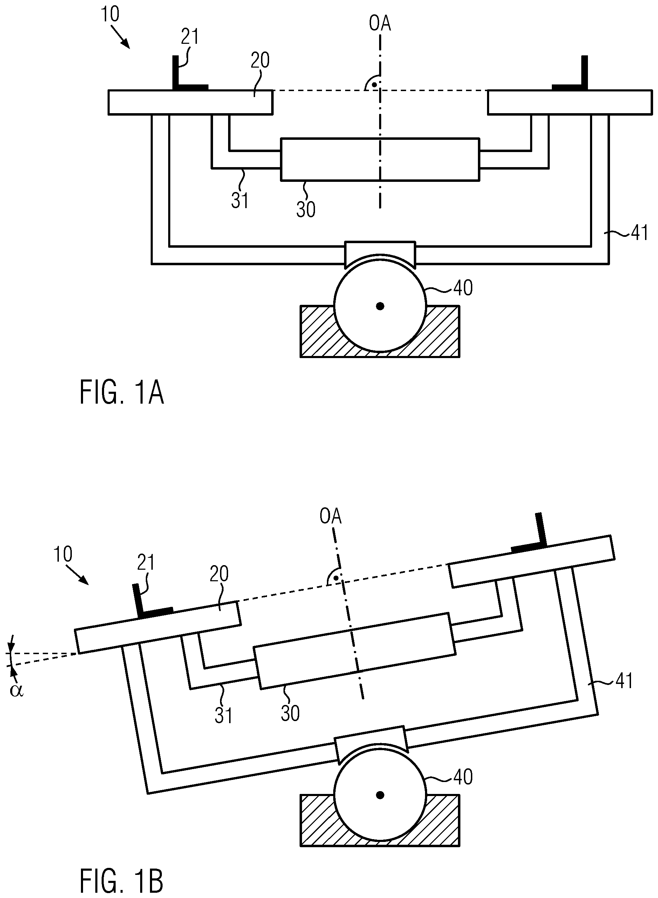

Figur 1A zeigt einen Querschnitt einer erfindungsgemäßen Kippvorrichtung 10 zur Mikroskopie gemäß einer ersten Ausführungsform. Dabei umfasst die Kippvorrichtung 10 einen Probenträgerhalter 20 und eine optische Komponente 30. Die optische Komponente 30 ist in diesem Beispiel lediglich schematisch dargestellt. Bei der optischen Komponente 30 kann es sich beispielsweise um einen Objektiv handeln, wobei das Objektiv eine Sammellinse oder ein Linsensystem umfassend eine Mehrzahl von Linsen sein kann. Die optische Komponente ist über eine Halterung 31 mit dem Probenträgerhalter 20 verbunden. Diese Verbindung ist in diesem Beispiel insbesondere starr, sodass keine relative Bewegung zwischen der optischen Komponente 30 und dem Probenträgerhalter 20 möglich ist.

Der Probenträgerhalter 20 umfasst zudem eine Einlegeschiene 21, in die ein Probenträger eingelegt werden und gegebenenfalls fixiert werden kann. Diese Einlegeschiene 21 kann umlaufend auf der oberen Seite des Probenträgerhalters 20 ausgebildet sein. Es ist ebenfalls möglich, dass die Einlegeschiene 21 lediglich an bestimmten Abschnitten auf der oberen Seite des Probenträgerhalters ausgebildet ist, beispielsweise sodass die Ecken des Probenträgers an der Einlegeschiene 21 anliegen. In diesem Beispiel lässt sich die Grundseite des Probenträgerhalters 20 als dessen obere Seite identifizieren, an der auch die Einlegeschiene 21 angebracht ist und auf der ein Probenträger eingelegt werden kann.

Der Probenträgerhalter 20 ist über eine Haltevorrichtung 41 mit einem Gelenk 40 verbunden und zugleich um dieses Gelenk 40 kippbar gelagert. Dabei kann es sich bei dem Gelenk 40 beispielsweise um ein Kugelgelenk oder ein Drehgelenk (Scharniergelenk) handeln. Die Kippachse verläuft im Fall eines Drehgelenks in die Zeichnungsebene und durch den Mittelpunkt des Gelenks 40. Für ein Kugelgelenk befindet sich die Kippachse entsprechend in einer horizontalen Ebene, die durch den Mittelpunkt des Gelenks 40 verläuft.

Zuletzt zeigt Figur 1A die optische Achse OA der optischen Komponente 30. Die optische Achse OA ist dabei senkrecht zur oben beschriebenen Grundseite des Probenträgerhalters 20 ausgerichtet und verläuft durch geometrische Zentrum und insbesondere durch das Krümmungszentrum der optischen Komponente 30. Zudem ist im gezeigten Beispiel die optische Achse OA senkrecht zur zuvor beschriebenen Kippachse ausgerichtet, die Ausrichtung bleibt in der Kippvorrichtung 10 stets erhalten.

Die beschriebene Kippvorrichtung aus Figur 1A ist in Figur 1B im gekippten Zustand gezeigt, wobei die Kippung um das Gelenk 40 und um eine Kippachse, die in die Zeichnungsebene hinein und durch den Mittelpunkt des Gelenks 40 verläuft, durchgeführt wurde. Da die optische Komponente 30 und der Probenträgerhalter 20 starr miteinander verbunden sind, wurden sie in der gleichen Weise gekippt, sodass insbesondere auch nach der Kippung die optische Achse OA der optischen Komponente senkrecht zur Grundseite des Probenträgerhalters 20 ausgerichtet ist.

Die Kippvorrichtung 10 wurde um den Winkel α gegenüber der horizontalen Ausrichtung der Kippvorrichtung, wie in Figur 1A, und um die beschriebene Kippachse gekippt. Die horizontale Ausrichtung kann als Ausgangsposition der Kippvorrichtung 10 betrachtet werden.

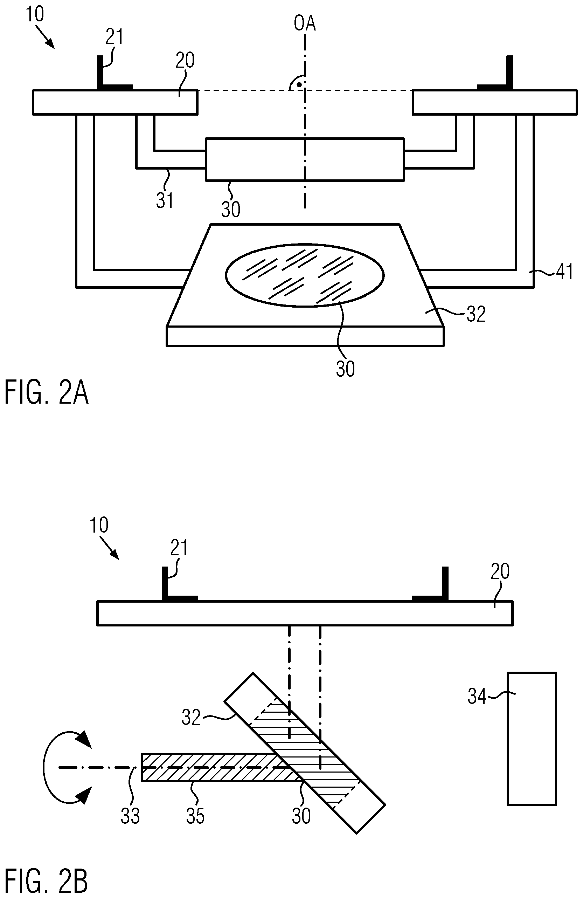

Eine weitere Ausführungsform einer Kippvorrichtung 10 ist in Figur 2A gezeigt. Diese Figur zeigt dabei einen Querschnitt der Kippvorrichtung 10 in Frontansicht. Dabei umfasst die Kippvorrichtung 10, wie bereits aus dem Ausführungsbeispiel gemäß Figur 1A bekannt, einen Probenträgerhalter 20 mit einer Einlegeschiene 21 und eine optische Komponente 30. In diesem Fall besteht die optische Komponente 30 aus einem Objektiv und einem Spiegel, wobei das Objektiv aus den zuvor genannten Gründen lediglich schematisch gezeichnet ist. Der Spiegel ist in einer Spiegelhalterung 32 gehaltert. In einer einfachen Form handelt es sich bei der Spiegelhalterung 32 um eine Platte mit einer Aussparung, wobei der Spiegel in dieser Aussparung gehaltert ist. Allerdings ist die Spiegelhalterung 32 nicht auf dieses Beispiel beschränkt.

Die Spiegelhalterung 32 ist gegenüber der Grundseite des Probenträgerhalters 20 gedreht, wobei die Rotationsachse des Spiegels und der Spiegelhalterung 32 parallel zur Grundseite des Probenträgerhalters 20 und in der Zeichenebene liegt. Insbesondere beträgt der Rotationswinkel 45 Grad, sodass Licht, das entlang der optischen Achse durch das Objektiv tritt, im rechten Winkel umgelenkt wird und senkrecht aus der Zeichenebene heraustritt.

Die Kippvorrichtung 10 ist insgesamt derart ausgebildet, dass eine Kippachse 33 an der Spiegelhalterung 32 angeordnet ist (nicht in dieser Figur, sondern in Figur 2B gezeigt) und zentral durch den Spiegel verläuft. Die Kippvorrichtung 10 ist also um diese Kippachse 33 drehbar gelagert. Die Kippachse 33 zeigt dabei senkrecht aus der Zeichenebene heraus und steht ebenso senkrecht zur optischen Achse OA. Die Kippvorrichtung 10 kann, wie durch die Pfeile dargestellt, im oder gegen den Uhrzeigersinn gekippt werden.

In dieser Ausführungsform der Kippvorrichtung 10 ist es möglich, eine Kippung durchzuführen, ohne dass sich der optische Pfad des Lichts nach der optischen Komponente 30 verändert. Dies liegt daran, dass das Zentrum der Rotation der Kippvorrichtung 10 an der Stelle des Spiegels liegt, wodurch Auftreffpunkt und Auftreffwinkel von Licht auf den Spiegel unabhängig von einer Kippung der Kippvorrichtung 10 konstant bleiben.

Eine Kippvorrichtung 10 gemäß einer weiteren Ausführungsform ist als Querschnitt und in einer Seitenansicht in Figur 2B dargestellt. Hierin bildet ein Spiegel allein die optische Komponente 30. Dabei ist der Spiegel in einer Spiegelhalterung 32 montiert und die Oberfläche des Spiegels ist gegenüber der Grundseite des Probenträgerhalters 20 rotiert, insbesondere um 45 Grad. Die Spiegelhalterung 32 umfasst weiterhin eine Kippachse 33, die, wie in Bezug auf Figur 2A beschrieben, senkrecht zur optischen Achse und zentral durch den Spiegel ausgerichtet ist. Zudem liegt die Kippachse 33 in dieser Seitenansicht in der Zeichenebene. Insbesondere ist die Anordnung der Kippachse 33 in den beiden Beispielen gemäß Figuren 2A und 2B identisch. Eine Kippung der Kippvorrichtung 10 wird durch eine Rotation um die Kippachse 33 erzeugt, beispielswiese um ein entsprechendes Gelenk 35, wobei die Rotationsrichtung durch die Pfeile dargestellt ist.

Das Objektiv 34 ist nicht Teil der Kippvorrichtung 10 und ist insbesondere fest montiert, sodass es sich unabhängig von einer Kippung der Kippvorrichtung 10 nicht bewegt. Diese Anordnung hat den Vorteil, dass das Objektiv, das zur einer Erzeugung eines Bilds von großer Bedeutung ist, nicht bewegt werden muss. Dadurch werden Abbildungsfehler, die durch eine relative Bewegung zwischen Spiegel und Objektiv induziert werden, unterdrückt. Das Objektiv 34 kann dabei insbesondere Bestandteil eines Mikroskops sein, insbesondere, wenn die Kippvorrichtung 10 in ein bestehendes Mikroskop integriert wird.

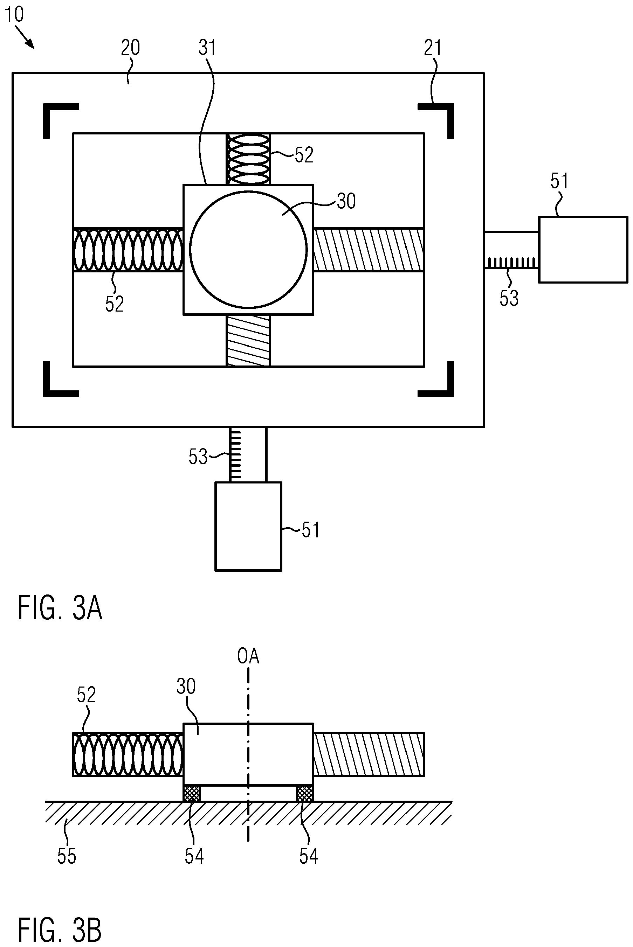

Figur 3A zeigt eine Kippvorrichtung 10 umfassend eine Bewegungseinrichtung 50 als Draufsicht, wobei die Bewegungseinrichtung 50 derart ausgebildet ist, dass sie die optische Komponente 30 gegenüber dem fixierten Probenträgerhalter 20 bewegt. Im konkreten Fall ist die Bewegungseinrichtung 50 eine Translationsplattform, in der die optische Komponente 30 in einer Ebene parallel zur Grundseite des Probenträgerhalters beweglich ist. Bei der Translationsplattform kann es sich um ein herkömmliches manuelles Modell handeln umfassend eine Stellschraube 51, eine Rückstellfeder 52 und eine ablesbare Skala 53 für jede der beiden Achsen. Durch eine Rotation der Stellschraube 51 kann die optische Komponente 30 entlang der zugehörigen Achse bewegt werden. Dadurch, dass zwei senkrecht zueinander angeordnete Achsen mit jeweils einer Stellschraube 51 vorhanden sind, die eine zweidimensionale Translationsplattform bilden, kann die optische Komponente 30 horizontal in einer Ebene und relativ zum Probenträgerhalter 20 bewegt werden.

Durch eine vergleichbare Form der Bewegungseinrichtung kann auch der Probenträgerhalter 20 bewegbar montiert sein und durch die Bewegungseinrichtung 50 bewegt werden, während die optische Komponente 30 fixiert ist.

Wie in den zuvor beschriebenen Ausführungsbeispielen umfasst die Kippvorrichtung weiterhin eine Einlegeschiene 21 auf der Oberseite des Probenträgerhalters 20, wobei die Einlegeschiene 21 für einen rechteckigen Probenträger konzipiert ist und den Probenträger an den vier Ecken fixiert, und eine Halterung 31 für die optische Komponente 30.

In Figur 3B ist eine weitere Ausführungsform für die Bewegungseinrichtung 50 gezeigt, mit der es möglich ist, die optische Komponente 30 zusätzlich parallel zu ihrer optischen Achse OA zu bewegen. Zum Ausführungsbeispiel in Figur 3A analoge Komponenten werden hier nicht erneut erläutert, sondern nur die Unterschiede werden hervorgehoben.

Der gezeigte Ausschnitt der Kippvorrichtung 10 umfasst eine Grundplatte 55, die fest in Bezug auf den Probenträgerhalter 20 angeordnet ist. Zwischen der Grundplatte 55 und der Halterung 31 der optischen Komponente sind symmetrisch Motoren 54 oder vergleichbare Antriebsvorrichtungen angeordnet, die eine vertikale Bewegung, entlang der optischen Achse OA der optischen Komponente 30 durchführen. Damit die optische Komponente 30 entlang der optischen Achse OA bewegt werden kann, müssen die beiden Motoren 54 synchron angetrieben werden. Beispielsweise kann es sich dabei um einen piezoelektrischen Aktor oder einen Elektromotor handeln. Insbesondere kann die hier gezeigte Bewegungseinrichtung mit jenem Ausführungsbeispiel aus Figur 3A kombiniert werden, sodass die Bewegungseinrichtung eine relative Bewegung zwischen dem Probenträgerhalter 20 und der optischen Komponente 30 entlang aller drei Raumrichtungen ermöglicht.

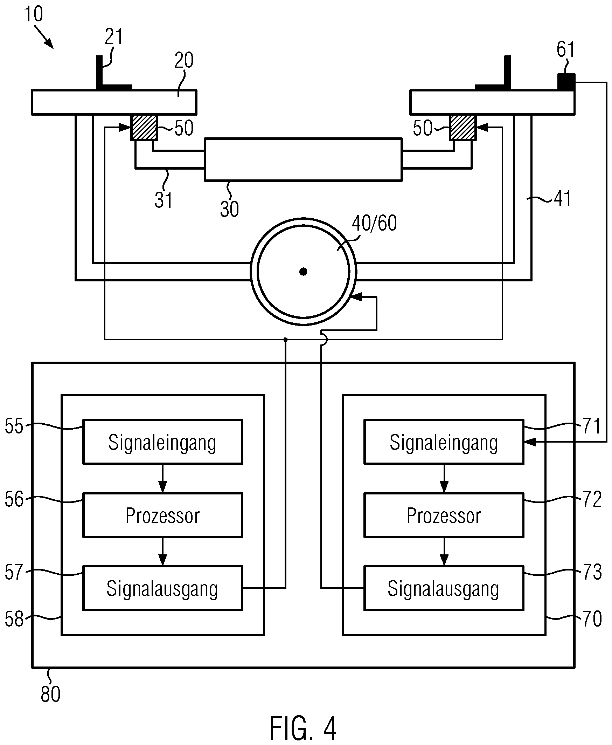

Eine Kippvorrichtung 10 gemäß einer weiteren Ausführungsform ist in Figur 4 gezeigt. Diese umfasst zunächst einige Komponenten des ersten Ausführungsbeispiels gemäß Figur 1A, sodass im Folgenden lediglich die Unterschiede erläutert werden.

Die gezeigte Kippvorrichtung 10 weist zum einen eine Bewegungseinrichtung 50 auf, die derart ausgebildet ist, dass sie die optische Komponente 30 in vertikaler Richtung, beziehungsweise entlang der optischen Achse OA bewegen kann. Man beachte, dass die optische Komponente 30 aufgrund der Konstruktion eine Kippung in der gleichen Weise, insbesondere um die gleiche Achse (oder eine koaxiale Achse) vollführen muss wie der Probenträgerhalter 20, aber die beiden Komponenten sind nicht starr miteinander verbunden, weil eine relative Bewegung zwischen den Komponenten stattfinden kann. Die Bewegungseinrichtung umfasst eine Halterung 31 für die optische Komponente 30 und einen Motor, beispielsweise einen Elektromotor oder einen piezoelektrischen Aktor, der die Bewegung der Bewegungseinrichtung 50 durchführen kann.

Die Bewegungseinrichtung 50 wird durch eine elektronische Steuerung 58 angesteuert, die die Bewegung des Motors regelt. Die elektronische Steuerung 58 umfasst hierbei einen Signaleingang 55, einen Prozessor 56 und einen Signalausgang 57. In den Signaleingang kann beispielsweise eine Zielkoordinate für die optische Komponente 30 eingegeben werden. Die Eingabe kann in einer zugehörigen Steuerungssoftware oder auch auf einer integrierten Tastatur oder einer ähnlichen Vorrichtung erfolgen. Diese Eingabe wird dann vom Prozessor 56 derart verwendet, dass daraus ein Steuerungssignal erzeugt wird. Dieses Steuerungssignal wird von der elektronischen Steuerung 58 durch den Signalausgang 57 ausgegeben und an die Bewegungseinrichtung 50 übertragen. Daraufhin bewegt die Bewegungseinrichtung 50 mittels des Motors die optische Komponente 30, sodass sie die vorgegebene Zielkoordinate einnimmt.

Ein Kippwinkel der Kippvorrichtung 10 kann über eine Neigungseinrichtung 60 gesteuert werden. Diese Neigungseinrichtung 60 kann beispielsweise ein, insbesondere elektrisch ansteuerbarer, Drehmotor sein. Dabei umfasst der Drehmotor gleichzeitig auch das Gelenk 40, um das die Kippung ausgeführt wird.

Weiterhin weist die Kippvorrichtung 10 einen Neigungssensor 61 auf, der den Kippwinkel der Kippvorrichtung 10 gegenüber einer horizontalen Ausgangsposition erfasst. Kapazitive oder magnetoresistive Flüssigkeitsneigungssensoren bieten sich beispielsweise an. Der Neigungssensor 61 gibt einen bestimmten Wert für den Kippwinkel oder einen anderen Messwert, der vom Kippwinkel abhängt, aus und überträgt diesen an die Steuerungseinrichtung 70.

Die Steuerungseinrichtung 70 umfasst ähnlich wie die elektrische Steuerung 58 einen Signaleingang 71, einen Prozessor 72 und einen Signalausgang 73. Der vom Neigungssensor 61 ausgegebene Wert wird in den Signaleingang 71 eingegeben. Über den Signaleingang 71 kann auch ein bestimmter Zielwert für den Kippwinkel eingegeben werden, auf den die Kippvorrichtung 10 eingestellt werden soll. Der Prozessor 72 verwendet diesen Wert und den vorgegebenen Zielwert und erzeugt daraus ein Steuerungssignal, das vom Signalausgang 73 ausgegeben und an die Neigungseinrichtung 60 übertragen wird. Im gezeigten Beispiel wird das Steuerungssignal direkt an den Drehmotor, als Komponente der Neigungseinrichtung 60, übertragen. Der Drehmotor empfängt das Steuerungssignal und erzeugt eine Kippung der Kippvorrichtung 10, sodass der Kippwinkel an den vorgegebenen Zielwert angepasst wird.

Die Steuerung des Kippwinkels kann insbesondere als Feedback-Schleife ausgebildet sein. Hierzu erfolgt eine kontinuierliche Regelung des Kippwinkels, indem der Neigungssensor 61 kontinuierlich einen Messwert ausgibt und die Steuerungseinrichtung 70 entsprechend kontinuierlich ein Steuerungssignal erzeugt und an die Neigungseinrichtung 60 überträgt, um die Kippvorrichtung 10 bei einem bestimmten Kippwinkel zu halten. Dadurch lässt sich der Kippwinkel über lange Zeiträume besonders stabil halten.

Die elektronische Steuerung 58 und die Steuerungseinrichtung 70 können von einer Kontrolleinrichtung 80 umfasst sein, die die gesamte beschriebene Steuerung der Kippvorrichtung durchführen kann. Dabei kann die Kontrolleinrichtung 80 mehrere Signaleingänge, Prozessoren und Signalausgänge, die mit den jeweiligen Einrichtungen der Kippvorrichtung 10 verbunden sein.

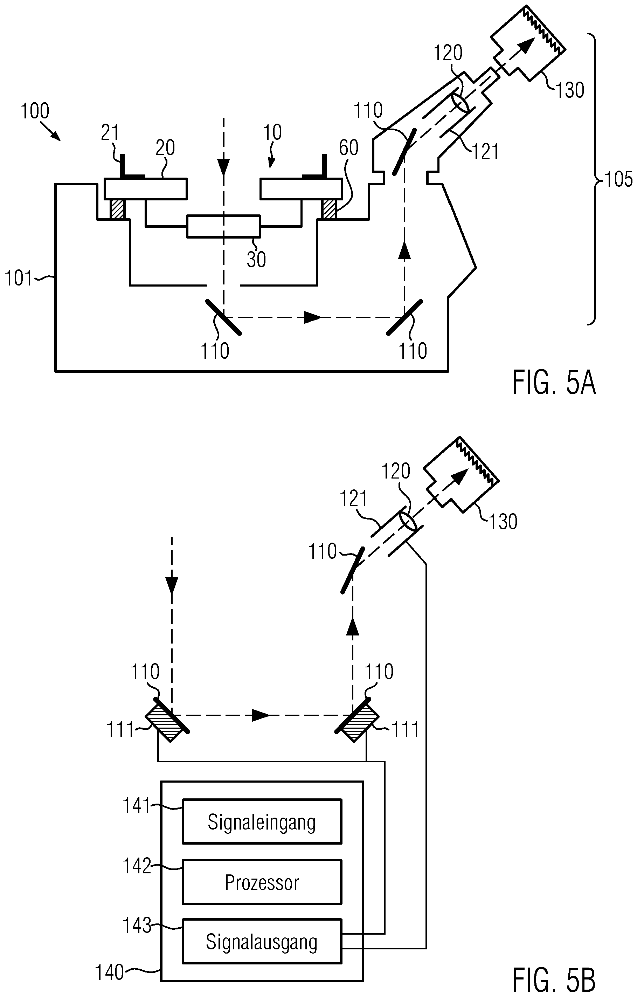

Figur 5A illustriert den schematischen Aufbau eines erfindungsgemäßen Mikroskops 100, im gezeigten Fall eines inversen Durchlichtmikroskops, im Querschnitt. Dabei umfasst das Mikroskop 100 eine beschriebene Kippvorrichtung 10 und ein Abbildungssystem 105. Als Halterung und stabilisierendes Element dient ein für ein derartiges Mikroskop übliches Stativ 101.

Das Mikroskop 100 kann grundsätzlich mit den in dieser Beschreibung erläuterten Kippvorrichtungen ausgestattet sein, sodass sie in dieser Figur lediglich auf die relevanten Komponenten reduziert, nämlich den Probenträgerhalter 20 mit einer Einlegeschiene 21, die optische Komponente 30 und eine Neigungseinrichtung 60, dargestellt ist. Die optische Komponente 30 kann insbesondere ein Objektiv sein, ist hier aber nur schematisch dargestellt.

Dabei kann das Mikroskop 100 modular aufgebaut sein, insbesondere kann die Kippvorrichtung 10 ein Austauschteil sein, das nachträglich in das Mikroskop 100 integriert werden kann. Hierbei können unterschiedliche Ausführungsformen der Kippvorrichtung 10 mit dem Mikroskop 100 kompatibel sein.

Das Abbildungssystem 105 umfasst mehrere Spiegel 110 und eine Abbildungslinse 120, die im vorliegenden Beispiel in Form eines Okulars ausgebildet ist. Die Abbildungslinse ist in einer Schiene 121 montiert, sodass die Abbildungslinse 120 entlang ihrer optischen Achse beweglich montiert ist. Genau wie bei der optischen Komponente 30 der Kippvorrichtung 10 verläuft auch die optische Achse der Abbildungslinse 120 durch das Krümmungszentrum und orthogonal zu den restlichen Symmetrieachsen. Weiterhin umfasst das Abbildungssystem 105 eine bildgebende Komponente 130 Bei der bildgebenden Komponente kann es sich beispielsweise um eine Kamera mit einem LCD-Chip oder einem anderen bildgebenden Sensor handeln, die an dem Mikroskop 100 montiert werden kann, um ein Bild elektronisch zu erzeugen und auszugeben.

Die Spiegel 110 dienen dazu, das Licht von der optischen Komponenten 30 durch die Abbildungslinse 120 auf die bildgebende Komponente 130 zu lenken und werden daher als Umlenkspiegel bezeichnet. Im gezeigten Beispiel werden hierfür drei Spiegel 110 verwendet, allerdings ist ein erfindungsgemäßes Mikroskop 100 nicht auf diese Anzahl und Konfiguration beschränkt. Zusätzlich können entlang des optischen Pfads noch weitere optische Elemente zur Strahlführung vorhanden sein, beispielsweise ein Prisma.

Ebenfalls in Figur 5A gezeigt ist der optische Pfad von Licht, das durch das Mikroskop 100 propagiert. Ausgehend von einer Lichtquelle oberhalb des Mikroskops (beispielsweise einem Kondensor, nicht dargestellt) tritt das Licht zunächst durch einen gegebenenfalls in den Probenträgerhalter 20 montierten Probenträger und danach durch die optische Komponente 30. Infolge einer Kippung der Kippvorrichtung 10 ändert sich die Lichtbrechung in der optischen Komponente 30 und infolge dessen auch der optische Pfad durch das Mikroskop 100. Daher verändern sich der Auftreffpunkt und der Auftreffwinkel auf die Abbildungslinse 120 und die bildgebende Komponente 130. Diese Veränderung kann durch die Spiegel 110 des Abbildungssystems 105 kompensiert werden. Dazu sind die Spiegel 110 bewegbar gelagert und können derart rotiert werden, dass besagter Auftreffpunkt und Auftreffwinkel unverändert bleiben. Dabei kann die Anpassung der Spiegel 110 manuell oder, wie nachfolgend erläutert, durch eine dafür vorgesehene Kontrolleinrichtung durchgeführt werden.

Falls sich die Länge des optischen Pfads stark ändert, kann es sein, dass ein vom der Abbildungslinse 120 abgebildetes Bild nicht mehr scharfgestellt ist. Für diesen Fall ist die Abbildungslinse 120 in einer Schiene 121 entlang ihrer optischen Achse bewegbar gelagert, sodass der Abstand zwischen der Abbildungslinse und der bildgebenden Komponente 130 angepasst werden und eine scharfe Abbildung erhalten werden kann. Alternativ kann das Objektiv auf unendlich eingestellt sein, sodass eine Veränderung der Länge des optischen Pfads zwischen dem Objektiv und der Abbildungslinse 120 zu keiner Verschiebung der Bildebene führt.

In Figur 5B ist eine Detailansicht des Mikroskops 100 aus 5A gargestellt, in der das Abbildungssystem 105 genauer erläutert wird. Zwei der enthaltenen Spiegel 110 umfassen jeweils einen Motor 111, mit dem die Spiegel 110 rotiert werden können, um eine beschriebene Veränderung des Auftreffpunkts und des Auftreffwinkels auf die Abbildungslinse 120 und die bildgebende Komponente 130 zu kompensieren. Dabei kann es sich um Spiegel handeln, die in einem herkömmlichen, piezogetriebenen Spiegelhalter ("*motorized mirror mount*") gehaltert sind, wie sie im Zusammenhang mit derartigen optischen Anwendungen geläufig sind.

Weiterhin ist die Abbildungslinse 120 in einer motorisierten Schiene 121 montiert, mit der die Abbildungslinse 120 entlang ihrer optischen Achse elektronisch bewegbar ist. Die Steuerung der Motoren 111 und der Schiene 121 erfolgt über eine elektronische Steuerungseinrichtung 140 umfassend einen Signaleingang 141, einen Prozessor 142 und einen Signalausgang 143. Der Signaleingang kann beispielsweise von der bildgebenden Komponente 130 bereitgestellt werden, die detektiert, dass ein Auftreffpunkt und/oder ein Auftreffwinkel auf die Komponente nicht mehr korrekt ist/sind und/oder dass eine Scharfstellung des Bildes nicht mehr gegeben ist. Das Signal kann dann zum Beispiel ein Fehlersignal sein, das die Abweichung des Auftreffwinkels und/oder des Auftreffpunktes widerspiegelt. Dieses Signal wird vom Signaleingang 141 empfangen und an den Prozessor 142 übertragen, der daraus eine notwendige Korrektur der Spiegelpositionen und/oder der Position der Abbildungslinse 120 errechnet. Dieses Korrektursignal wird vom Signalausgang 143 ausgegeben und an die Motoren 111 und/oder die Schiene 121 übertragen, wo die entsprechende Korrektur des optischen Pfads und/oder des Brennpunkts der Abbildungslinse 120 durchgeführt werden. Die Steuerungseinrichtung 140 kann insbesondere mit einem Feedback-Mechanismus ausgestattet sein, sodass die beschriebene Korrektur kontinuierlich stattfinden kann. Zudem kann die Steuerungseinrichtung 140 Teil der externen Kontrolleinrichtung 80 sein, also darin integriert sein.

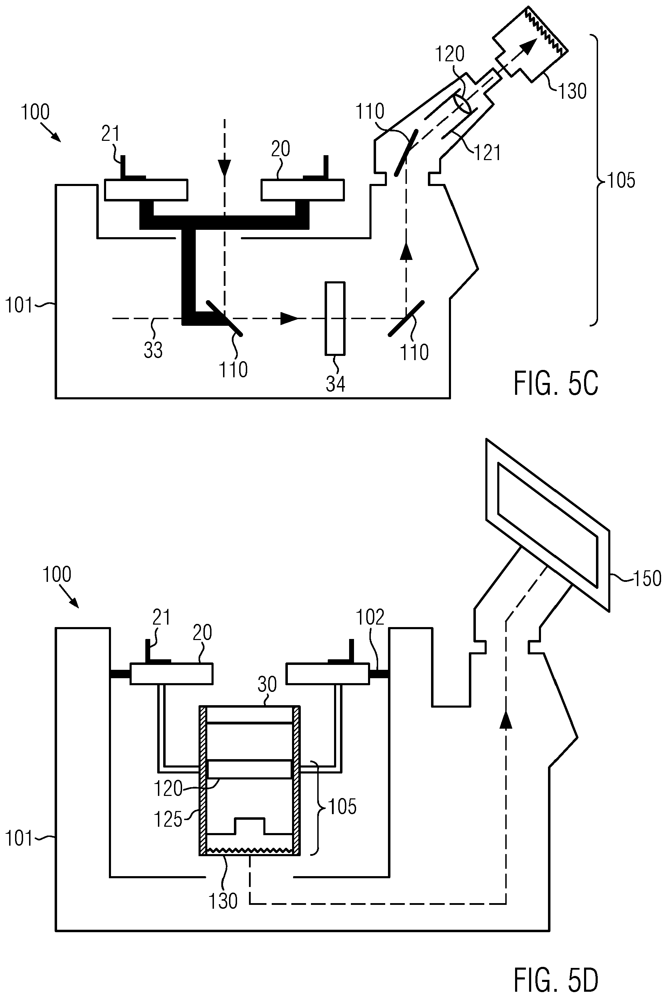

Figur 5C zeigt ein erfindungsgemäßes Mikroskop 100 gemäß einer weiteren Ausführungsform. Dieses unterscheidet sich von der Ausführungsform der Figur 5A durch die Ausführung der Kippvorrichtung 10, der optischen Komponente 30 und des Abbildungssystems 105. Auf diese Unterschiede wird im Folgenden genauer eingegangen.

Zunächst wird eine Kippvorrichtung 10, wie in Figur 2B gezeigt, verwendet. Dabei umfasst die Kippvorrichtung 10 einen Spiegel als optische Komponente 30. Der Spiegel ist mittels einer entsprechenden Vorrichtung mit dem Probenträgerhalter 20 verbunden, insbesondere starr verbunden. Die Kippachse 33 des Spiegels und damit auch der gesamten Kippvorrichtung 10 ist derart ausgerichtet, dass sie parallel zur Grundseite des Probenträgerhalters 20 und durch das geometrische Zentrum des Spiegels verläuft. Eine Kippung der Kippvorrichtung 10 erfolgt dann um die besagte Kippachse 33.

Wie bereits oben beschrieben, erlaubt es die Ausführungsform der Kippvorrichtung 10, dass sich trotz einer Kippung der optische Pfad des Lichts nach einer Reflexion von dem Spiegel nicht verändert. Daher ist es möglich, die Kippvorrichtung mit einem im Mikroskop 100 integrierten Objektiv 34 zu kombinieren. Insbesondere muss es im Fall einer Kippung nicht nachjustiert werden, was eine besonders effiziente Mikroskopie erlaubt. Dabei kann das Objektiv 34 grundsätzlich bewegbar montiert sein, um beispielsweise dessen Position entlang des optischen Pfads anzupassen, sodass anfangs einer mikroskopischen Untersuchung ein scharfes Bild erzeugt wird.

Das Abbildungssystem umfasst zwei Umlenkspiegel 110, eine bewegbar in einer Schiene montierte Abbildungslinse 120 und eine bildgebende Komponente 130. Insbesondere hinsichtlich der Abbildungslinse 120 und der bildgebenden Komponente 130 gelten die gleichen Erwägungen wie im vorhergehenden Ausführungsbeispiel der Figur 5A.

Ein erfindungsgemäßes Mikroskop 100 gemäß einer weiteren Ausführungsform wird mit Bezug auf Figur 5D erläutert. Darin umfasst das Mikroskop 100 ebenfalls eine beschriebene Kippvorrichtung 10, die an dieser Stelle nicht mehr näher erläutert werden muss, und ein Stativ 101. Weiterhin umfasst das Mikroskop 100 ein Abbildungssystem 105 mit einer Abbildungslinse 120 und einer bildgebende Komponente 130, insbesondere einer Kamera mit einem CCD-Chip oder gleichartigen Vorrichtung.

Der Probenträgerhalter 20 ist über eine Halterung 102 mit dem Stativ 101 verbunden und in Bezug auf das Stativ 101 drehbar oder kippbar gelagert. Die Drehung, beziehungsweise Kippung, erfolgt um eine Achse, die koaxial zur Halterung 102 ausgerichtet ist.

Die optische Komponente 30, die Abbildungslinse 120 und die bildgebende Komponente 130 sind durch ein insbesondere umfangsgeschlossenes Tubusrohr 125 miteinander verbunden. Die Verbindung ist dabei derart, dass sich die drei genannten Komponenten nicht relativ zueinander bewegen können. Dabei erfüllt das Tubusrohr 125 nicht nur die Funktion einer stabilen Halterung, sondern verhindert auch, dass Licht, beispielsweise Streulicht, seitlich in die bildgebende Komponente 130 gelangen kann. Dadurch wird eine hohe Abbildungsqualität gewährleistet.

Das Tubusrohr 125 ist mit dem Probenträgerhalter 20 verbunden, sodass bei einer Kippung das gesamte System aus Kippvorrichtung 10 und Abbildungssystem 105 eine gemeinsame Kippbewegung vollführen, ändert sich der optische Pfad durch das System infolge einer Kippung nicht. Korrekturen am optischen Pfad, beispielsweise mithilfe von Umlenkspiegeln, sind daher nicht erforderlich.

Das Objektiv 30 kann alternativ entlang der vertikalen Achse beweglich angebracht sein, um den Abstand zwischen dem Objektiv 30 und dem Probenträgerhalter 20 zu verändern. Dabei kann das Objektiv 30 insbesondere auf unendlich eingestellt werden, sodass die Abbildungslinse 120 nicht an eine veränderte Position des Objektivs 30 angepasst werden muss.

Hinsichtlich der Implementierung einer Kippung gelten die zuvor gemachten Ausführungen. Beispielsweise kann die Kippung motorisiert stattfinden, wobei zum Beispiel die Halterung 102 durch einen entsprechend ausgebildeten Motor rotiert werden kann. Gleiche Erwägungen treffen auch auf eine mögliche Bewegbarkeit des Objektivs zu. Auch diese kann durch einen entsprechend ausgebildeten Motor implementiert werden. Es ist weiterhin möglich, dass die vom Mikroskop 100 umfasste Kippvorrichtung eine Bewegungseinrichtung mit den zuvor genannten Eigenschaften umfasst.

Die bildgebende Komponente 130 ist mit einer Anzeigevorrichtung 150, beispielsweise einem Monitor, der Teil des Mikroskops 100 sein kann, insbesondere auf elektronischem Wege verbunden, um ein elektronisches Signal von der bildgebenden Komponente 130 an die Anzeigevorrichtung 150 zu übertragen. Damit werden die von der bildgebenden Komponente 130 erzeugten Bilder von der Anzeigevorrichtung 150 einem Benutzer angezeigt.

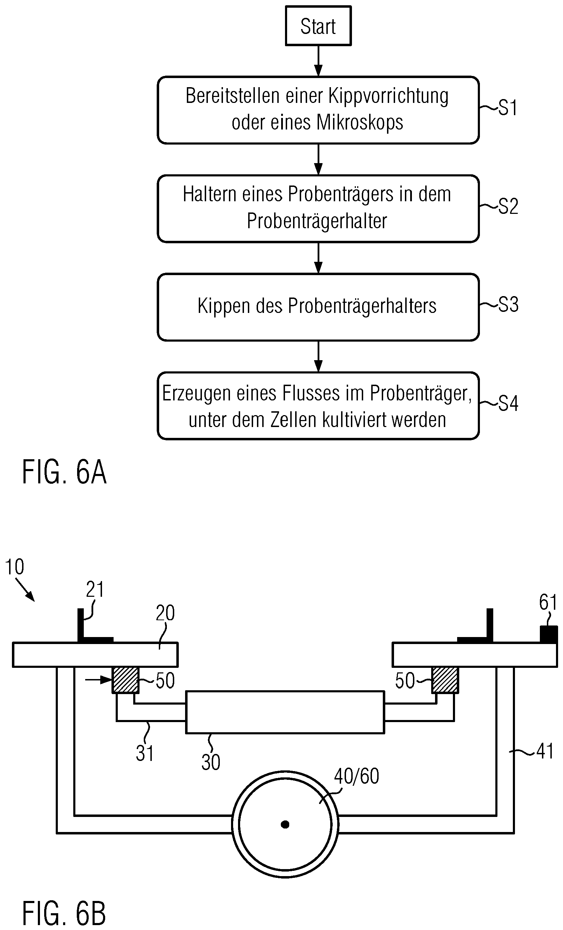

Figur 6A zeigt ein erfindungsgemäßes Verfahren zur Mikroskopie als Flussdiagramm. Im ersten Schritt S1 des Verfahrens wird eine hier beschriebene Kippvorrichtung 10 oder ein hier beschriebenes Mikroskop 100 bereitgestellt. Das Verfahren kann dabei grundsätzlich mit allen hierin vorgestellten Ausführungsformen durchgeführt werden.

Im nachfolgenden Schritt S2 wird ein Probenträger 200 in dem Probenträgerhalter 20 der Kippvorrichtung 10 gehaltert. Der Probenträger 200 umfasst dabei ein erstes Reservoir, ein zweites Reservoir und einen Kanal, der das erste Reservoir mit dem zweiten Reservoir verbindet. In dem Probenträger 200 befinden sich eine Flüssigkeit und Zellen oder eine Zellstruktur, wobei die Zellen ein einem Reservoir, einem Kanal oder an mehreren Stellen im Probenträger verteilt sein können.

Nun wird in Schritt S3 der Probenträgerhalter 20 gekippt, wobei für die Kippung grundsätzlich keine Einschränkungen hinsichtlich des Kippwinkels und/oder der Kippachse vorgesehen sind. Insbesondere werden Kippungen um einen Winkel zwischen 0 Grad und 20 Grad durchgeführt. Diese Kippung erzeugt durch Gravitation einen Fluss infolge eines Flüssigkeitstransports vom ersten Reservoir durch den Kanal in das zweite Reservoir.

Unter diesem Fluss werden die Zellen im Probenträger 200 kultiviert (Schritt S4).

Das zuvor beschriebene Verfahren zur Mikroskopie wird detaillierter mit Bezug auf die Figuren 6B bis 6D illustriert.

Im ersten Schritt des Verfahrens wird, wie in Figur 6B gezeigt, eine zuvor beschriebene Kippvorrichtung 10 bereitgestellt. Dabei ist die Kippvorrichtung 10 im Querschnitt als Frontalansicht gezeigt. Das Verfahren kann allerdings in der gleichen Art und Weise auch mit einem beschriebenen Mikroskop 100 umfassend die Kippvorrichtung 10 durchgeführt werden. Erfindungsgemäß umfasst die Kippvorrichtung 10 einen Probenträgerhalter 20 und eine optische Komponente 30. Darüber hinaus weisen der Probenträgerhalter 20 eine Einlegeschiene 21 und die optische Komponente 30 eine Halterung 31 auf, die den Probenträgerhalter 20 und die optische Komponente 30 starr miteinander verbindet. Gemäß dem zuvor beschriebenen Ausführungsbeispiel umfasst die Kippvorrichtung 10 weiterhin eine Bewegungseinrichtung 50 und eine Neigungseinrichtung 60, die gleichzeitig als Gelenk 40, um das eine Kippung erfolgt, dient. Für eine genauere Erläuterung der Komponenten wird auf das zugehörige Ausführungsbeispiel in Figur 4 verwiesen.

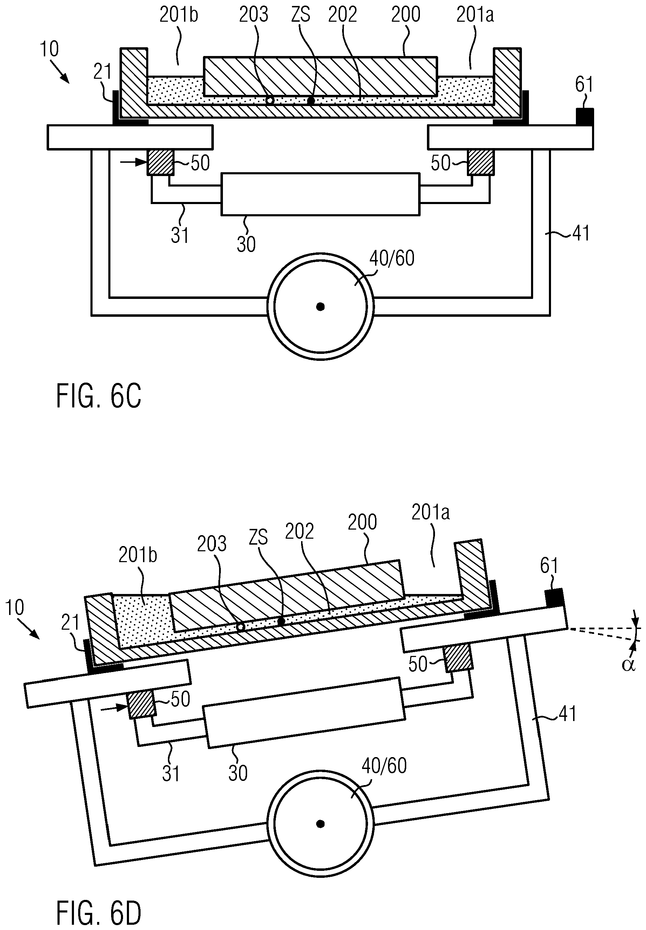

Im folgenden Verfahrensschritt (siehe Figur 6C) wird nun ein Probenträger 200 in den Probenträgerhalter 20 eingebracht und darin gehaltert. Dazu wird der Probenträger 200 in die Einlegeschiene 21 eingelegt, die genau auf die Größe des Probenträgers angepasst sein kann. Um darüber hinaus zu verhindern, dass sich der Probenträger innerhalb der Einlegeschiene 21 oder in dem Probenträgerhalter 20 im Allgemeinen bewegen kann, können weitere Befestigungseinrichtungen vorhanden sein. Hierzu bietet sich beispielsweise eine Feststellschraube an, die seitlich durch die Einlegeschiene 21 führt und den Probenträger 200 gegen die Einlegeschiene 21 drückt und dadurch fixiert. Dies kann von Vorteil sein, weil der Probenträger 200 in einem nicht ideal angepassten Probenträger 20 infolge einer Kippung leicht verrutschen kann und die mikroskopische Untersuchung dadurch erheblich beeinträchtigt wird.

Der Probenträger selbst umfasst ein erstes Reservoir 201a, ein zweites Reservoir 201b und einen Kanal 202, der das erste Reservoir 201a mit dem zweiten Reservoir 201b verbindet. Die Bezeichnungen erstes Reservoir 201a und zweites Reservoir 201b können austauschbar verwendet werden, da sie sich strukturell nicht voneinander unterscheiden. Im Probenträger 200 befinden sich eine Flüssigkeit, insbesondere ein Zellmedium, sowie Zellen oder Zellstrukturen ZS. Diese können als Teil des Verfahrens in den Probenträger 200 eingebracht werden oder bereits im Probenträger 200 vorhanden sein. Die Zellen selbst können sich wie gezeigt an einer bestimmten Stelle innerhalb des Kanals 202 befinden, allerdings können sie auch in einem Reservoire 201a, 201b angeordnet sein oder über größeren Bereich innerhalb des Probenträgers 200 verteilt sein.

Zudem umfasst der Probenträger einen Flüssigkeitssensor 203, der in diesem Beispiel innerhalb des Kanals 202 angeordnet ist und derart ausgebildet ist, eine Flussgeschwindigkeit innerhalb des Kanals zu erfassen. Der ermittelte Wert kann dann elektronisch ausgegeben werden.

Anschließend wird wie in Figur 6D gezeigt die Kippvorrichtung 10 und damit auch der Probenträgerhalter 20 und die optische Komponente 30 um einen Winkel α gekippt. Zur Durchführung der Kippung wird eine Neigungseinrichtung 60 wie im zuvor beschriebenen Ausführungsbeispiel einer Kippvorrichtung 10 eingesetzt.

Infolge der Kippung wird die Flüssigkeit vom ersten Reservoir 201a in Richtung des zweiten Reservoirs 201b transportiert. Durch diesen Flüssigkeitstransport entsteht ein Fluss, insbesondere auch am Ort der Zellen, wobei die Zellen unter diesem Fluss kultiviert werden. Hierbei erlaubt die Kippvorrichtung 10, wie beschrieben, eine präzise Einstellung und Kontrolle des Kippwinkels und damit des Flusses. Hierzu kann insbesondere der Flüssigkeitssensor 203 verwendet werden, der einen zugehörigen Messwert an die Steuerungseinrichtung 70 übertragt. Aus der gemessenen Flussgeschwindigkeit kann der Prozessor einen Zielwert für den Kippwinkel und daraus ein Steuerungssignal ermitteln, der entsprechend an die Neigungseinrichtung 60 übertragen wird. Die Neigungseinrichtung stellt damit den Kippwinkel entsprechend ein. Dieses Verfahren kann in der gleichen Weise auch mit einem gemessenen Flüssigkeitsstand oder einer gemessenen Flussrichtung durchgeführt werden, wobei der Flüssigkeitssensor 203 entsprechend ausgebildet sein muss.

Das beschriebene Verfahren kann über die beschriebenen Schritte hinaus erweitert werden, indem der Kippwinkel zeitlich variiert wird. Dazu muss die Neigungseinrichtung 60 derart ausgebildet sein, dass sie eine kontinuierliche Anpassung des Kippwinkels α vornehmen kann. Beispielsweise kann der Kippwinkel linear oder periodisch, wie zum Beispiel sinusförmig, mit der Zeit geändert werden. Insbesondere erfolgt dabei ein Vorzeichenwechsel des Kippwinkels, als eine Änderung der Kipprichtung, sodass die Flussrichtung innerhalb des Probenträgers 200 umgekehrt wird.

Durch dieses beschriebene Verfahren kann ein kontinuierlicher Fluss des Zellmediums in dem Probenträger 200 erzeugt werden, ohne dass dafür externe Vorrichtungen benötigt werden, weil die Flüssigkeit durch periodisches Umkehren der Kipprichtung kontinuierlich in Bewegung gehalten werden kann. Weiterhin kann der Kippwinkel kontinuierlich kontrolliert und eingestellt werden, beispielsweise mithilfe eine Feedback-Mechanismus der Neigungseinrichtung 60 im Zusammenspiel mit einem Flüssigkeitssensor und einem Neigungssensor 61.

Das beschriebene Verfahren ist nicht auf die gezeigte Kombination einer Kippvorrichtung 10 und einer Probenträgers 200 beschränkt. Insbesondere ist das Verfahren mit allen in dieser Beschreibung vorgestellten Kippvorrichtungen 10 und Mikroskopen 100 durchführbar. Gleichsam ist auch die Merkmalskombination der Probenträgers 200 nicht einschränkend. So kommen für das Verfahren Probenträger infrage, sofern sie zwei durch einen Kanal 202 verbundene Reservoire 201a, 201b aufweisen und sinnvoll mit der Kippvorrichtung 10 oder dem Mikroskop 100 verwendet werden können. Der Probenträger 200 kann beispielsweise neben den erwähnten Komponenten zusätzlich Mikrofilterstrukturen oder Ventile aufweisen.

## Patentansprüche

1. Kippvorrichtung (10) zur Mikroskopie, umfassend:
einen Probenträgerhalter (20),
eine optische Komponente (30) mit einer optischen Achse (OA), und
eine Neigungseinrichtung (60) mit einem Motor (13),
wobei die optische Achse (OA) zu einer Grundseite des Probenträgerhalters (20) ausgerichtet ist,
wobei der Probenträgerhalter (20) und die optische Komponente (30) kippbar gelagert sind,
wobei die Ausrichtung der optischen Achse (OA) bezüglich der Grundseite des Probenträgerhalters (20) erhalten bleibt, wenn der Probenträgerhalter (20) und die optische Komponente (30) gekippt werden,
wobei der Motor (13) mit der optischen Komponente (30) und/oder dem Probenträgerhalter (20) verbunden ist,
wobei der Motor (13) derart ausgebildet ist, dass er den Probenträgerhalter (20) und die optische Komponente (30) kippt, und
wobei die Neigungseinrichtung (60) derart ausgebildet ist, dass sie eine kontinuierliche Kippbewegung, insbesondere eine wippende Bewegung, der Kippvorrichtung (10) erzeugt.

2. Kippvorrichtung (10) nach Anspruch 1, wobei es sich bei der optischen Komponente (30) um ein Objektiv und/oder einen Spiegel handelt.

3. Kippvorrichtung (10) nach einem der vorangegangenen Ansprüche, wobei der Probenträgerhalter (20) und die optische Komponente (30) jeweils mit einem Gelenk (40) verbunden sind und um das jeweilige Gelenk (40) kippbar gelagert sind.

4. Kippvorrichtung (10) nach einem der vorangegangenen Ansprüche, wobei die Kippvorrichtung (10) derart ausgebildet ist, dass der Probenträgerhalter (20) und die optische Komponente (30) um eine Kippachse (33) gekippt werden, die senkrecht zur optischen Achse (OA) ausgerichtet ist.

5. Kippvorrichtung (10) nach einem der vorangegangenen Ansprüche, wobei der Probenträgerhalter (20) und die optische Komponente (30) bezüglich einer horizontalen Position des Probenträgerhalters (20) um einen Kippwinkel zwischen 0 Grad und 1 Grad, insbesondere zwischen 0 Grad und 20 Grad gekippt werden können.

6. Kippvorrichtung (10) nach einem der vorangegangenen Ansprüche, weiterhin umfassend eine Bewegungseinrichtung (50),
wobei die Bewegungseinrichtung derart ausgebildet ist, dass sie die optische Komponente (30) in einer Ebene parallel zur Grundseite des Probenträgerhalters (20) bewegen kann, oder
wobei die Bewegungseinrichtung (50) derart ausgebildet ist, dass sie die optische Komponente (30) senkrecht zur Grundseite des Probenträgerhalters (20), beziehungsweise parallel zur optischen Achse (OA), bewegen kann.

7. Kippvorrichtung nach Anspruch 6, wobei die Bewegungseinrichtung (50) derart ausgebildet ist, dass sie den Probenträgerhalter (20) in einer Ebene senkrecht zur optischen Achse (OA) der optischen Komponente (30) bewegen kann, oder
wobei die Bewegungseinrichtung (50) derart ausgebildet ist, dass sie den Probenträgerhalter (20) parallel zur optischen Achse (OA) bewegen kann.

8. Kippvorrichtung nach Anspruch 7, weiterhin umfassend:
einen Neigungssensor (61), und
eine Steuerungseinrichtung (70),
wobei der Neigungssensor (61) derart ausgebildet ist, dass er einen Wert eines Kippwinkels der Kippvorrichtung (10) erfassen und ausgeben kann, und
wobei die Steuerungseinrichtung (70) derart ausgebildet ist, den von dem Neigungssensor (61) ausgegebenen Wert zu empfangen und aufgrund des empfangenen Werts und eines vorgegebenen Zielwerts ein Steuerungssignal an die Neigungseinrichtung (60) zu übertragen, sodass die Neigungseinrichtung (60) den Kippwinkel der Kippvorrichtung (10) auf den vorgegebenen Zielwert einstellt.

9. Mikroskop (100), umfassend,
eine Kippvorrichtung (10) nach einem der Ansprüche 1 bis 8,
ein Stativ (101), und
ein Abbildungssystem (105) mit einer Abbildungslinse (120), insbesondere einem Okular.

10. Mikroskop (100) nach Anspruch 9, wobei das Abbildungssystem (105) weiterhin umfasst:
einen oder mehrere Spiegel (110), wobei die Spiegel (110) mechanisch gekoppelt sind oder individuell elektronisch ansteuerbar sind, und/oder
eine bildgebende Komponente (130), beispielsweise eine Kamera mit einem bildgebenden Sensor.

11. Mikroskop (100) nach einem der Ansprüche 9 oder 10,
wobei die Abbildungslinse (120) entlang ihrer optischen Achse beweglich montiert ist, und/oder wobei eine Brennweite der Abbildungslinse (120) veränderlich ist, und
wobei eine Bewegung der Abbildungslinse (120) entlang ihrer optischen Achse und/oder die Brennweite individuell steuerbar sind.

12. Verfahren zur Mikroskopie, umfassend:
Bereitstellen einer Kippvorrichtung (10) zur Mikroskopie nach einem der Ansprüche 1 bis 9 oder eines Mikroskops (100) nach einem der Ansprüche 9 bis 11;
Haltern eines Probenträgers (200) in dem Probenträgerhalter (20); und
Kippen des Probenträgerhalters (20),
wobei der Probenträger (200) ein erstes Reservoir (201a), ein zweites Reservoir (201b) und einen Kanal (202) umfasst,
wobei das erste Reservoir (201a) über den Kanal (202) mit dem zweiten Reservoir (201b) verbunden ist,
wobei durch das Kippen des Probenträgerhalters (20) eine Flüssigkeit vom ersten Reservoir (201a) in das zweite Reservoir (201b) transportiert wird,
wobei insbesondere durch den Transport der Flüssigkeit ein Fluss entsteht, und wobei Zellen (ZS) unter diesem Fluss kultiviert werden.

13. Verfahren nach Anspruch 12,
wobei eine Kippvorrichtung (10) nach Anspruch 8 oder 9 bereitgestellt wird, und
wobei der Probenträger (200) weiterhin einen Flüssigkeitssensor umfasst,
wobei der Flüssigkeitssensor (203) einen Wert eines oder mehrerer von einem Flüssigkeitsstand, einer Flussgeschwindigkeit oder einer Flussrichtung innerhalb des in dem Probenträgerhalter (20) gehalterten Probenträgers (200) erfasst und ausgibt, und
wobei die Steuerungseinrichtung (70) den von dem Flüssigkeitssensor (203) ausgegebenen Wert empfängt und aufgrund des empfangenen Werts ein Steuerungssignal an die Neigungseinrichtung (60) überträgt, sodass die Neigungseinrichtung (60) den Kippwinkel der Kippvorrichtung (10) einstellt, sodass eines oder mehrere von dem Flüssigkeitsstand, der Flussgeschwindigkeit oder der Flussrichtung innerhalb des Probenträgers (200) an einen vordefinierten Zielwert angepasst werden.

14. Verwendung einer Kippvorrichtung (10) zur Mikroskopie nach einem der Ansprüche 1 bis 8 oder eines Mikroskops (100) nach einem der Ansprüche 9 bis 11 zum Transportieren einer Flüssigkeit innerhalb eines Probenträgers (200),
wobei der Probenträger (200) in dem Probenträgerhalter (20) gehaltert ist,
wobei der Probenträger (200) ein erstes Reservoir (201a), ein zweites Reservoir (201b) und einen Kanal (202) umfasst,
wobei das erste Reservoir (201a) über den Kanal (202) mit dem zweiten Reservoir (201b) verbunden ist, und
wobei durch Kippen des Probenträgerhalters (20) eine Flüssigkeit vom ersten Reservoir (201a) in das zweite Reservoir (201b) transportiert wird,
wobei insbesondere durch den Transport der Flüssigkeit ein Fluss entsteht, und wobei Zellen (ZS) unter diesem Fluss kultiviert werden.

## Claims

1. Tilting device (10) for microscopy, comprising:
a sample carrier mount (20),
an optical component (30) with an optical axis (OA), and
an inclination unit (60) with a motor (13),
where said optical axis (OA) is aligned with respect to a base side of said sample carrier mount (20),
where said sample carrier mount (20) and said optical component (30) are mounted to be tiltable, and
where the alignment of said optical axis (OA) with respect to said base side of said sample carrier mount (20) is maintained when said sample carrier mount (20) and said optical component (30) are tilted,
where said motor (13) is connected to said optical component (30) and/or to said sample carrier mount (20),
where said motor (13) is configured such that it tilts said sample carrier mount (20) and said optical component (30), and
where the inclination unit (60) is configured such that it generates a continuous tilt motion, in particular a seesaw motion, of the tilting device (10).

2. Tilting device (10) according to claim 1, where said optical component (30) is an objective and/or a mirror.

3. Tilting device (10) according to one of the preceding claims, where said sample carrier mount (20) and said optical component (30) are each connected to a joint (40) and are mounted to be tiltable about said respective joint (40).

4. Tilting device (10) according to one of the preceding claims, where said tilting mechanism (10) is configured such that said sample carrier mount (20) and said optical component (30) are tilted about a tilt axis (33) which is aligned to be perpendicular to said optical axis (OA).

5. Tilting device (10) according to one of the preceding claims, where said sample carrier mount (20) and said optical component (30) can be tilted by a tilt angle between 0 degrees and 1 degree, in particular between 0 degrees and 20 degrees, with respect to a horizontal position of said sample carrier mount (20).

6. Tilting device (10) according to one of the preceding claims, furthermore comprising a motion device (50),
where said motion unit (50) is configured such that it can move said optical component (30) in a plane parallel to said base side of said sample carrier mount (20), or
where said motion unit (50) is configured such that it can move said optical component (30) perpendicular to said base side of said sample carrier mount (20), or parallel to said optical axis (OA), respectively.

7. Tilting mechanism according to claim 6, where said motion unit (50) is configured such that it can move said sample carrier mount (20) in a plane perpendicular to said optical axis (OA) of said optical component (30), or
where said motion device (50) is configured such that it can move said sample carrier mount (20) parallel to said optical axis (OA).

8. Tilting device (10) according to claim 7, furthermore comprising:
an inclination sensor (61), and
a control unit (70),
where said inclination sensor (61) is configured such that it can detect and output a value of a tilt angle of said tilting mechanism (10), and
where said control unit (70) is configured to receive the value output by said inclination sensor (61) and, based on the value received and a predetermined target value, to transmit a control signal to said inclination unit (60) so that said inclination unit (60) adjusts the tilt angle of said tilting mechanism (10) to said predetermined target value.

9. Microscope (100) comprising,
a tilting device (10) according to one of the claims 1 to 8,
a stand (101), and
a projection system (105) with a projection lens (120), in particular an eyepiece.

10. Microscope (100) according to claim 9, where said projection system (105) furthermore comprises:
one or more mirrors (110), where said mirrors (110) are mechanically coupled or controllable electronically individually, and/or
an imaging component (130), for example a camera with an imaging sensor.

11. Microscope (100) according to one of the claims 9 or 10,
where said projection lens (120) is mounted to be movable along its optical axis, and/or where a focal length of said projection lens (120) is variable, and
where a motion of said projection lens (120) along its optical axis and/or said focal length can be controlled individually.

12. Method for microscopy comprising:
providing a tilting device (10) for microscopy according to one of the claims 1 to 9 or a microscope (100) according to one of the claims 9 to 11;
mounting a sample carrier (200) in said sample carrier mount (20); and
tilting said sample carrier mount (20),
where said sample carrier (200) comprises a first reservoir (201a), a second reservoir (201b), and a channel (202),
where said first reservoir (201a) is connected to said second reservoir (201b) by way of said channel (200),
where a fluid is transported from said first reservoir (201a) into said second reservoir (201b) by tilting said sample carrier mount (20),
where a flow is produced in particular by the transport of said fluid, and where cells (ZS) are cultured under this flow.

13. Method according to claim 12,
where a tilting device (10) according to claim 8 or 9 is provided, and
where said sample carrier (200) furthermore comprises a fluid sensor (203),
where said fluid sensor (203) detects and outputs a value of one or more of a fluid level, a flow rate, or a flow direction within said sample carrier (200) mounted in said sample carrier mount (20), and
where said control unit (70) receives the value that is output by said fluid sensor (203) and, based on the value received, transmits a control signal to said inclination unit (60), so that said inclination device (60) adjusts the tilt angle of said tilting device (10) so that one or more of the fluid level, the flow rate, or the flow direction within said sample carrier (200) are adjusted to a predefined target value.

14. Use of a tilting device (10) for microscopy according to one of the claims 1 to 8 or a microscope (100) according to one of the claims 9 to 11 for transporting a fluid within a sample carrier (200),
where said sample carrier (200) is mounted in said sample carrier mount (20),
where said sample carrier (200) comprises a first reservoir (201a), a second reservoir (201b), and a channel (202),
where said first reservoir (201a) is connected to said second reservoir (201b) by way of said channel (200), and
where a fluid is transported from said first reservoir (201a) into said second reservoir (201b) by tilting said sample carrier mount (20),
where a flow is produced in particular by the transport of the fluid, and where cells (ZS) are cultured under this flow.

## Revendications

1. Dispositif de basculement (10) pour la microscopie, comprenant :
un support de porte-échantillon (20),
un composant optique (30) avec un axe optique (OA), et
un dispositif d'inclinaison (60) avec un moteur (13),
dans lequel l'axe optique (OA) est aligné avec un côté de base du support de porte-échantillon (20),
dans lequel le support de porte-échantillon (20) et le composant optique (30) sont montés de manière inclinable,
dans lequel l'orientation de l'axe optique (OA) par rapport au côté de base du support de porte-échantillon (20) est maintenue lorsque le support de porte-échantillon (20) et le composant optique (30) sont inclinés,
dans lequel le moteur (13) est connecté au composant optique (30) et/ou au support de porte-échantillon (20),
dans lequel le moteur (13) est conçu de manière à incliner le support de porte-échantillon (20) et le composant optique (30), et
dans lequel le dispositif d'inclinaison (60) est conçu de manière à générer un mouvement d'inclinaison continu, en particulier un mouvement de bascule, du dispositif de basculement (10).

2. Dispositif de basculement (10) selon la revendication 1, dans lequel le composant optique (30) est un objectif et/ou un miroir.

3. Dispositif de basculement (10) selon l'une des revendications précédentes, dans lequel le support de porte-échantillon (20) et le composant optique (30) sont chacun connectés à une articulation (40) et sont montés de manière à pouvoir être inclinés autour de l'articulation (40) respective.

4. Dispositif de basculement (10) selon l'une des revendications précédentes, dans lequel le dispositif de basculement (10) est conçu de manière à ce que le support de porte-échantillon (20) et le composant optique (30) soient basculés autour d'un axe d'inclinaison (33) qui est aligné perpendiculairement à l'axe optique (OA).

5. Dispositif de basculement (10) selon l'une des revendications précédentes, dans lequel le support de porte-échantillon (20) et le composant optique (30) peuvent être inclinés par rapport à une position horizontale du support de porte-échantillon (20) d'un angle d'inclinaison compris entre 0 degré et 1 degré, en particulier entre 0 degré et 20 degrés.

6. Dispositif de basculement (10) selon l'une des revendications précédentes, comprenant en outre un dispositif de mouvement (50),
dans lequel le dispositif de mouvement est conçu de manière à pouvoir déplacer le composant optique (30) dans un plan parallèle au côté de base du support de porte-échantillon (20), ou
dans lequel le dispositif de mouvement (50) est conçu de manière à pouvoir déplacer le composant optique (30) perpendiculairement au côté de base du support de porte-échantillon (20), ou parallèlement à l'axe optique (OA).

7. Dispositif de basculement selon la revendication 6, dans lequel le dispositif de mouvement (50) est conçu de manière à pouvoir déplacer le support de porte-échantillon (20) dans un plan perpendiculaire à l'axe optique (OA) du composant optique (30), ou
dans lequel le dispositif de mouvement (50) est conçu de manière à pouvoir déplacer le support de porte-échantillon (20) parallèlement à l'axe optique (OA).

8. Dispositif de basculement selon la revendication 7, comprenant en outre :
un capteur d'inclinaison (61), et
un dispositif de commande (70),
dans lequel le capteur d'inclinaison (61) est conçu de manière à pouvoir détecter et émettre une valeur d'un angle d'inclinaison du dispositif de basculement (10), et
dans lequel le dispositif de commande (70) est conçu pour recevoir la valeur émise par le capteur d'inclinaison (61) et pour transmettre un signal de commande au dispositif d'inclinaison (60) sur la base de la valeur reçue et d'une valeur cible prédéterminée, de sorte que le dispositif d'inclinaison (60) ajuste l'angle d'inclinaison du dispositif de basculement (10) à la valeur cible prédéterminée.

9. Microscope (100), comprenant,
un dispositif de basculement (10) selon l'une quelconque des revendications 1 à 8,
un socle (101), et
un système d'imagerie (105) avec une lentille d'imagerie (120), en particulier un oculaire.

10. Microscope (100) selon la revendication 9, dans lequel le système d'imagerie (105) comprend en outre :
un ou plusieurs miroirs (110), les miroirs (110) étant couplés mécaniquement ou pouvant être commandés électroniquement individuellement, et/ou
un composant d'imagerie (130), par exemple une caméra avec un capteur d'imagerie.

11. Microscope (100) selon l'une quelconque des revendications 9 ou 10,
dans lequel la lentille d'imagerie (120) est montée de manière mobile le long de son axe optique,
et/ou dans lequel une longueur focale de la lentille d'imagerie (120) est variable, et
dans lequel un mouvement de la lentille d'imagerie (120) le long de son axe optique et/ou la longueur focale peuvent être commandés individuellement.

12. Procédé de microscopie, comprenant :
la fourniture d'un dispositif de basculement (10) pour la microscopie selon l'une quelconque des revendications 1 à 9 ou un microscope (100) selon l'une quelconque des revendications 9 à 11 ;
le maintien d'un porte-échantillon (200) dans le support de porte-échantillon (20) ; et
l'inclinaison du support de porte-échantillon (20),
dans lequel le porte-échantillon (200) comprend un premier réservoir (201a), un deuxième réservoir (201b) et un canal (202),
dans lequel le premier réservoir (201a) est connecté au deuxième réservoir (201b) *via* le canal (202),
dans lequel un liquide est transporté du premier réservoir (201a) dans le deuxième réservoir (201b) par inclinaison du support de porte-échantillon (20),
un écoulement est créé en particulier par le transport du liquide, et les cellules (ZS) sont cultivées sous cet écoulement.

13. Procédé selon la revendication 12,
dans lequel un dispositif de basculement (10) selon la revendication 8 ou 9 est fourni, et
dans lequel le porte-échantillon (200) comprend en outre un capteur de liquide,
dans lequel le capteur de liquide (203) détecte et émet une valeur d'un ou plusieurs éléments parmi un niveau de liquide, une vitesse d'écoulement ou une direction d'écoulement à l'intérieur du porte-échantillon (200) maintenu dans le support de porte-échantillon (20), et
dans lequel le dispositif de commande (70) reçoit la valeur émise par le capteur de liquide (203) et transmet un signal de commande au dispositif d'inclinaison (60) sur la base de la valeur reçue, de sorte que le dispositif d'inclinaison (60) ajuste l'angle d'inclinaison du dispositif de basculement (10) de sorte qu'un ou plusieurs du niveau de liquide, de l'angle d'inclinaison et du niveau de liquide du dispositif de basculement (10) soient égalisés,
la vitesse ou la direction de l'écoulement à l'intérieur du porte-échantillon (200) peut être ajustée à une valeur cible prédéfinie.

14. Utilisation d'un dispositif de basculement (10) pour la microscopie selon l'une quelconque des revendications 1 à 8 ou d'un microscope (100) selon l'une quelconque des revendications 9 à 11 pour transporter un liquide à l'intérieur d'un porte-échantillon (200),
dans lequel le porte-échantillon (200) est maintenu dans le support de porte-échantillon (20),
dans lequel le porte-échantillon (200) comprend un premier réservoir (201a), un deuxième réservoir (201b) et un canal (202),
dans lequel le premier réservoir (201a) est connecté au deuxième réservoir (201b) *via* le canal (202), et
dans lequel un liquide est transporté du premier réservoir (201a) dans le deuxième réservoir (201b) par inclinaison du support de porte-échantillon (20),
un flux est écoulement, notamment par le transport du liquide, et des cellules (ZS) sont cultivées sous cet écoulement.
